# EUROPEAN PATENT SPECIFICATION

(11) **EP 4 509 611 B1**
(45) Date of publication and mention of the grant of the patent: **02.09.2026**
(21) Application number: 24186915.5
(22) Date of filing: 05.07.2024
(51) Int. Cl.: C12P 19/02, C07H 1/06, C12N 9/10, C12P 19/12, C12P 19/18, C12P 19/24, C13K 5/00, C13B 20/14, C13B 20/16, C13B 30/02, C12N 9/90

(54) **A SIMPLE METHOD FOR ANIMAL FREE LACTOSE SYNTHESIS**
EINFACHES VERFAHREN ZUR TIERFREIEN SYNTHESE VON LAKTOSE
PROCÉDÉ SIMPLE DE SYNTHÈSE DE LACTOSE SANS ANIMAL

(30) Priority: 07.07.2023 EP 23184324
(43) Date of publication of application: 19.02.2025
(73) Proprietor: Inbiose N.V., 9052 Zwijnaarde (BE)
(72) Inventor: BEAUPREZ, Joeri, 9052 Zwijnaarde (BE); VERHAEGHE, Tom, 9052 Zwijnaarde (BE)
(74) Representative: Saelens, Claire

(56) References cited:
- WO-A1-2015/150328
- US-A1- 2020 148 793
- SADOVNIKOVA ANNA ET AL: "A Comparative Review of the Cell Biology, Biochemistry, and Genetics of Lactose Synthesis", JOURNAL OF MAMMARY GLAND BIOLOGY AND NEOPLASIA, PLENUM PRESS, NEW YORK, NY, US, vol. 26, no. 2, 1 June 2021 (2021-06-01), pages 181 - 196, XP037490184, ISSN: 1083-3021, [retrieved on 20210614], DOI: 10.1007/S10911-021-09490-7
- SEUNG-KYUNG CHUNG ET AL: "Characterization of UDP-glucose 4-epimerase from Pyrococcus horikoshii: Regeneration of UDP to produce UDP-galactose using two-enzyme system with trehalose", BIORESOURCE TECHNOLOGY, vol. 110, 1 April 2012 (2012-04-01), AMSTERDAM, NL, pages 423 - 429, XP055263014, ISSN: 0960-8524, DOI: 10.1016/j.biortech.2012.01.046
- CATALDI T R ET AL: "Carbohydrate analysis by high-performance anion-exchange chromatography with pulsed amperometric detection: The potential is still growing", FRESENIUS J ANAL CHEM, 1 December 2000 (2000-12-01), XP093237029, Retrieved from the Internet <URL:https://link.springer.com/article/10.1007/s002160000588> [retrieved on 20250106]
- HARPER W J: "LACTOSE AND LACTOSE DERIVATIVES", WHEY AND LACTOSE PROCESSING, XX, XX, 1 January 1992 (1992-01-01), pages 317 - 360, XP009022754

## Description

The present invention relates to methods to produce lactose, more specifically without the need for isolation from mammalian milk. Furthermore, the present invention provides for methods to purify the produced lactose.

Lactose as a disaccharide knows a wide variety of applications, in the food and pharmaceutical industry (Hebbink GA, Dickhoff BHJ. Chapter 5 - Application of lactose in the pharmaceutical industry. In: Paques M, Lindner CBT-L, editors. Academic Press; 2019. p. 175-229. Available from: https://www.sciencedirect.com/science/article/pii/B9780128117200000052; Hettinga KA. Chapter 6 - Lactose in the dairy production chain. In: Paques M, Lindner CBT-L, editors. Academic Press; 2019. p. 231-66. Available from: https://www.sciencedirect.com/science/article/pii/B9780128117200000064). Today it is isolated from mammalian milk, mainly cow's milk. Due to the environmental impact of this production process, there is a need for alternative synthesis methods that can compete with the well-established extraction methods from milk at low production cost.

Mammals synthesize lactose by means of a lactose synthase, or beta-1,4 galactosyltransferase, transferring a galactose to glucose with UDP-galactose as a donor substrate and glucose as an acceptor substrate. This principle has been engineered in microbes, copying the mammalian biosynthesis pathway (Mao Z, Shin H-D, Chen RR. Engineering the E. coli UDP-glucose synthesis pathway for oligosaccharide synthesis. Biotechnol Prog [Internet]. Jan [cited 2016 Mar 13];22(2):369-74. Available from: http://www.ncbi.nlm.nih.gov/pubmed/16599548) and the synthesis enzymes have been applied in vitro before to synthesize lactose by the addition of the expensive UDP-galactose (Lau K, Thon V, Yu H, Ding L, Chen Y, Muthana MM, et al. Highly efficient chemoenzymatic synthesis of [small beta]1-4-linked galactosides with promiscuous bacterial [small beta]1-4-galactosyltransferases. Chem Commun [Internet]. 2010;46(33):6066-8. Available from: http://dx.doi.org/10.1039/C0CC01381A) as donor.

A challenge one encounters with these methodologies is the energy requirement for lactose synthesis. The synthesis of UDP-galactose over UDP-glucose requires UTP, which either has to be added to the reaction mixture or be synthesized by the microbial host. After lactose synthesis UDP is released which is either lost as a substrate or may be recycled into UTP by means of ATP, another costly biochemical intermediate. Furthermore, the addition or synthesis of galactose is required, which is currently still derived from lactose and no alternative synthesis methods have been identified.

Purely from an energy point and cost point of view, the current state of the art would not be able to compete with extractive production of lactose, even if it is more environmentally friendly.

### Summary of the invention

It is an object of the present invention to provide for methods which solve the problem of the energy requirement for lactose synthesis and the production cost. The present invention provides a new method for producing lactose wherein we solved the problem of stoichiometric addition of nucleotides and galactose.

According to the invention, this and other objects are achieved by providing a method for the production of lactose, characterised in that said method comprises use of i) a synthase and ii) UDP, for forming UDP-glucose.

The method can further comprise the use of an UDP-glucose-4-epimerase on the UDP-glucose which results in the synthesis of UDP-galactose which is then used by a galactosyltransferase to form lactose. More specifically a beta 1,4-galactosyltransferase is used to form lactose with glucose as an acceptor. In a preferred method, the glucose is also produced from the carbon source, preferably sucrose, by the addition of a glucose isomerase, which converts fructose into glucose. In an alternative preferred method, the glucose is fed together with sucrose. This method allows the full conversion of sucrose or a combined feed of sucrose and glucose into lactose without the addition of stoichiometric amounts of nucleotides.

Key intermediates in the process from sucrose to lactose include UDP-glucose, UDP-galactose and glucose. Key intermediates in the process from a combined feed of sucrose and glucose, to lactose include UDP-glucose and UDP-galactose.

The present invention further also provides efficient methods for purification of the produced lactose. Further benefits of the teachings of this invention will be apparent to one skilled in the art from reading this invention.

### Definitions

The words used in this specification to describe the invention and its various embodiments are to be understood not only in the sense of their commonly defined meanings, but to include by special definition in this specification structure, material or acts beyond the scope of the commonly defined meanings. Thus, if an element can be understood in the context of this specification as including more than one meaning, then its use in a claim must be understood as being generic to all possible meanings supported by the specification and by the word itself.

The various aspects and embodiments of the invention disclosed herein are to be understood not only in the order and context specifically described in this specification, but to include any order and any combination thereof. Each embodiment as identified herein may be combined together unless otherwise indicated. All publications, patents, and patent applications mentioned in this specification are herein incorporated by reference to the same extent as if each individual publication, patent, or patent application was specifically and individually indicated to be incorporated by reference in its entirety. Unless specifically stated otherwise, all words used in the singular number shall be deemed to include the plural and vice versa. Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described herein are those well-known and commonly employed in the art. Standard techniques are used for nucleic acid and peptide synthesis. Generally, enzymatic reactions and purification steps are performed according to the manufacturer's specifications.

In the specification, there have been disclosed embodiments of the invention, and although specific terms are employed, the terms are used in a descriptive sense only and not for purposes of limitation, the scope of the invention being set forth in the following claims. It must be understood that the illustrated embodiments have been set forth only for the purposes of example and that it should not be taken as limiting the invention. It will be apparent to those skilled in the art that alterations, other embodiments, improvements, details and uses can be made consistent with the letter and spirit of the disclosure herein and within the scope of this disclosure, which is limited only by the claims, construed in accordance with the patent law, including the doctrine of equivalents. In the claims that follow, reference characters used to designate claim steps are provided for convenience of description only, and are not intended to imply any particular order for performing the steps, unless specifically stated otherwise.

Throughout the application, unless explicitly stated otherwise, the features "synthesize", "synthesized" and "synthesis" are interchangeably used with the features "produce", "produced" and "production", respectively. Throughout the application, unless explicitly stated otherwise, the expressions "capable of...<verb>" and "capable to...<verb>" are preferably replaced with the active voice of said verb and *vice versa.* For example, the expression "capable of expressing" is preferably replaced with "expresses" and *vice versa,* i.e., "expresses" is preferably replaced with "capable of expressing". Throughout this document and in its claims, the verbs "to comprise", "to have" and "to contain" and their conjugations are used in their non-limiting sense to mean that items following the word are included, but items not specifically mentioned are not excluded. The verb "to consist essentially of" means that a solution or a composition as defined herein may comprise additional component(s) than the ones specifically identified, said additional component(s) not altering the unique characteristic of the invention. Said additional compound(s) might be inevitable by-product(s), for example, generated during production of lactose of present invention as well as compound(s) that were introduced into a process stream from which lactose is recovered but which could not have been removed therefrom. The term "consisting essentially of" with respect to spray-dried powders includes spray-dried powders containing with respect to the dry matter of the spray-dried powder at least 80 %-wt., at least 85 %-wt., at least 90 % -wt., at least 93 %-wt., at least 95 %-wt. or at least 98 %-wt. of lactose. The term "consisting essentially of" is used likewise with respect to spray-dried powders, process streams and solutions containing lactose. Throughout this document and in its claims, unless specifically stated otherwise, the verbs "to comprise", "to have" and "to contain", and their conjugations, may be preferably replaced by "to consist of" (and its conjugations) or "to consist essentially of" (and its conjugations) and vice versa. In addition, reference to an element by the indefinite article "a" or "an" does not exclude the possibility that more than one of the elements is present, unless the context clearly requires that there be one and only one of the elements. The indefinite article "a" or "an" thus usually means "at least one". Throughout the document and in the claims, unless explicitly stated otherwise, the articles "a" and "an" are preferably replaced by "at least one", more preferably "at least two", even more preferably by "at least three", even more preferably by "at least four", even more preferably by "at least five", even more preferably by "at least six", most preferably by "at least two". The word "about" or "approximately" when used in association with a numerical value (e.g., "about 10") or with a range (e.g., "about x to approximately y") preferably means that the value or range is interpreted as being as accurate as the method used to measure it. If no error margins are specified, the expression "about" or "approximately" when used in association with a numerical value is interpreted as having the same round-off as the given value. Throughout this document and its claims, unless otherwise stated, the expression "from x to y", wherein x and y represent numerical values, refers to a range of numerical values wherein x is the lower value of the range and y is the upper value of the range. Herein, x and y are also included in the range.

Throughout the application, unless explicitly stated otherwise, the term "beta 1,4-galactosyltransferase" is to be understood to refer to an enzyme being able to transfer a galactose from UDP-galactose to glucose to form lactose. Such beta-1,4- galactosyltransferase is preferably a lactose synthase. An example of such galactosyltransferase is the N-acetylglucosamine beta-1,4-galactosyltransferase GalT (Uniprot ID Q51116, sequence version 2023_02 of 03 May 2023) from *Neisseria meningitidis.*

The term "bioconversion" is to be understood as a conversion of a product by resting or permeabilized cells.

Throughout the application, unless explicitly stated otherwise, the terms "UDP-glucose-4-epimerase" and "UDP-galactose-4-epimerase" are used interchangeably.

The terms "glucose isomerase", "xylose isomerase" and "fructose isomerase" are used in the art to refer to an enzyme converting fructose to glucose and vice versa.

Protein or polypeptide sequence information and functional information can be provided by a comprehensive resource for protein sequence and annotation data like e.g., the Universal Protein Resource (UniProt) (www.uniprot.org) (Nucleic Acids Res. 2021, 49(D1), D480-D489). UniProt comprises the expertly and richly curated protein database called the UniProt Knowledgebase (UniProtKB), together with the UniProt Reference Clusters (UniRef) and the UniProt Archive (UniParc). The UniProt identifiers (UniProt ID) are unique for each protein present in the database. Throughout the application, the sequence of a polypeptide is represented by an UniProt ID. Unless stated otherwise, the UniProt IDs of the proteins described correspond to their sequence version 01 as present in the UniProt Database (www.uniprot.org) version release 2023_02 of 03 May 2023.

It should be understood for those skilled in the art that for the databases used herein, the content of each database is fixed at each release and is not to be changed. When the content of a specific database is changed, this specific database receives a new release version with a new release date. All release versions for each database with their corresponding release dates and specific content as annotated at these specific release dates are available and known to those skilled in the art.

### Brief description of the drawings

Figure 1 shows production of lactose using sucrose as raw material or carbon source.
Figure 2 shows production of lactose using trehalose as raw material or carbon source.
Figure 3 shows production of lactose using sucrose and glucose as raw materials or carbon source.

### Detailed description of the invention

In a first aspect, the present invention provides a method for the production of lactose, wherein the method comprises use of i) a synthase and ii) Uridine Diphoshate (UDP), for forming UDP-glucose. According to the invention, the synthase is a sucrose synthase and said synthase forms fructose.

UDP is preferably added in catalytic amounts, more preferably said UDP is provided in a concentration between 0,1 mM and 10 mM. Alternatively, UDP is preferably added in stoichiometric amounts. Preferably, the method according to the present invention further comprises use of UDP-glucose-4-epimerase to convert said UDP-glucose to UDP-galactose.

Alternatively or preferably, said UDP-galactose is a galactose donor for a beta 1,4-galactosyltransferase to form lactose.

Alternatively or preferably, the method can further comprise use of a beta 1,4-galactosyltransferase to convert glucose with UDP-galactose to form lactose and UDP. Preferably, said beta 1,4-galactosyltransferase uses glucose as an acceptor and UDP-galactose as a donor.

Preferably, the UDP released by the beta 1,4-galactosyltransferase is recycled to be used by the synthase to form UDP-glucose. The UDP generated can thus in turn be used in sucrose synthase catalyzed reactions.

In a preferred embodiment of the present invention, in the methods wherein the synthase also forms a fructose, said fructose is converted to glucose.

Preferably said sucrose synthase is chosen from the GT4 glycosyltransferase subfamily, or chosen from KEGG Enzyme class EC 2.4.1.13. Alternatively, said sucrose synthase is chosen from the list of the sucrose synthase (Uniprot ID P13708, sequence version 2023_02 of 03 May 2023) from *Glycine max,* the sucrose synthase (Uniprot ID P49040, sequence version 2023_02 of 03 May 2023) from *Arabidopsis thaliana,* the sucrose synthase (Uniprot ID Q9ZEV2, sequence version 2023_02 of 03 May 2023) from *Anabaena* sp. PCC 7119 (ATCC no. 29151), the sucrose synthase (Uniprot ID A0A059ZV61, sequence version 2023_02 of 03 May 2023) from *Acidithiobacillus caldus* ATCC 51756 or any protein sequence comprising a sequence identity of 80% or more to any one of the sucrose synthase (Uniprot ID P13708, sequence version 2023_02 of 03 May 2023) from *Glycine max,* the sucrose synthase (Uniprot ID P49040, sequence version 2023_02 of 03 May 2023) from *Arabidopsis thaliana,* the sucrose synthase (Uniprot ID Q9ZEV2, sequence version 2023_02 of 03 May 2023) from *Anabaena* sp. PCC 7119 (ATCC no. 29151), the sucrose synthase (Uniprot ID A0A059ZV61, sequence version 2023_02 of 03 May 2023) from *Acidithiobacillus caldus* ATCC 51756 and having sucrose synthase activity.

Preferably said UDP-glucose-4-epimerase is selected from enzyme class EC 5.1.3.2, or selected from the list of GalE (Uniprot ID P09147, sequence version 2023_02 of 03 May 2023) from *Escherichia coli* K-12 MG1655, GalE (Uniprot ID E8MF10, sequence version 2023_02 of 03 May 2023) from *Bifidobacterium longum* subsp. *longum,* GalE (Uniprot ID A0A0K1E1Q5, sequence version 2023_02 of 03 May 2023) from *Pyroboculum sp. WP30,* GalE (Uniprot ID Q0P9C3, sequence version 2023_02 of 03 May 2023) from *Campylobacter jejuni* subsp. *jejuni* serotype O:2, GalE (UniProt ID P26503 sequence version 2023_02 of 03 May 2023) from *Sinorhizobium meliloti* (strain 1021) or any protein sequence comprising a sequence identity of 80% or more to any one of GalE (Uniprot ID P09147, sequence version 2023_02 of 03 May 2023) from *Escherichia coli* K-12 MG1655, GalE (Uniprot ID E8MF10, sequence version 2023_02 of 03 May 2023) from *Bifidobacterium longum* subsp. *longum,* GalE (Uniprot ID A0A0K1E1Q5, sequence version 2023_02 of 03 May 2023) from *Pyrobaculum sp. WP30,* GalE (Uniprot ID Q0P9C3, sequence version 2023_02 of 03 May 2023) from *Campylobacter jejuni* subsp. jejuni serotype O:2, GalE (UniProt ID P26503 sequence version 2023_02 of 03 May 2023) from *Sinorhizobium meliloti* (strain 1021) and having UDP-glucsose-4-epimerase activity.

Preferably said beta 1,4-galactosyltransferase is a lactose synthase or selected from Enzyme Class EC 2.4.1.87, GalT (Uniprot ID Q51116, sequence version 2023_02 of 03 May 2023) from *Neisseria meningitidis,* or GalT (UniProt ID D0EAD4 sequence version 2023_02 of 03 May 2023) of *Pasteurella multocida* M1404 or any protein with a sequence comprising a sequence identity of 80% or more to said GalT (Uniprot ID Q51116, sequence version 2023_02 of 03 May 2023) from *Neisseria meningitidis* or to said GalT (UniProt ID D0EAD4 sequence version 2023_02 of 03 May 2023) of *Pasteurella multocida* M1404.

Preferably said glucose isomerase is selected from the list of XylA (Uniprot ID P37031, sequence version 2023_02 of 03 May 2023) from *Streptomyces murinus,* XylA (Uniprot ID P12070, sequence version 2023_02 of 03 May 2023) from *Arthrobacter* sp. (strain NRRL B3728), XylA (Uniprot ID D2DK62, sequence version 2023_02 of 03 May 2023) from *Thermoanaerobacter ethanolicus,* XylA (Uniprot ID P00944 sequence version 2023_02 of 03 May 2023) from *Escherichia coli* K-12 MG1655 or any protein with a sequence comprising a sequence identity of 80% or more to any one of said XylA (Uniprot ID P37031, sequence version 2023_02 of 03 May 2023) from *Streptomyces murinus,* XylA (Uniprot ID P12070, sequence version 2023_02 of 03 May 2023) from *Arthrobacter* sp. (strain NRRL B3728), XylA (Uniprot ID D2DK62, sequence version 2023_02 of 03 May 2023) from *Thermoanaerobacter ethanolicus,* XylA (Uniprot ID P00944 sequence version 2023_02 of 03 May 2023) from *Escherichia coli* K-12 MG1655 and having glucose isomerase activity.

In a preferred embodiment of the present invention the method is a fermentation process, a bioconversion, or an enzymatic process. The fermentation process used in the present invention can be a microbial fermentation process or a cell culture fermentation process.

Cells used in such fermentation or bioconversion process are selected from the list consisting of a microorganism, a plant cell, an animal cell, an insect cell or a protozoan cell, more preferably said cell is a microorganism, more preferably said cell is a bacterium or a yeast, even more preferably said cell is a bacterium, even more preferably said cell is a bacterium belonging to the genus of *Escherichia* or *Bacillus,* even more preferably said cell is a bacterium belonging to the genus of *Escherichia,* even more preferably said cell is *Escherichia coli,* even more preferably said cell is an *Escherichia coli* K-12 strain, most preferably said cell is *Escherichia coli* MG1655.

Throughout the application and claims, unless specifically stated otherwise, a microorganism is preferably a bacterium, a yeast or a fungus, more preferably a bacterium or a yeast, most preferably a bacterium. Throughout the application and claims, unless specifically stated otherwise, a bacterium preferably belongs to the phylum of the Proteobacteria or the phylum of the Firmicutes or the phylum of the Cyanobacteria or the phylum Deinococcus-Thermus. Said bacterium belonging to the phylum Proteobacteria belongs preferably to the family Enterobacteriaceae, preferably to the species *Escherichia coli.* Said bacterium preferably relates to any strain belonging to the species *Escherichia coli* such as but not limited to *Escherichia coli* B, *Escherichia coli* C, *Escherichia coli* W, *Escherichia coli* K12, *Escherichia coli* Nissle. More specifically, said bacterium relates to cultivated *Escherichia coli* strains - designated as *E. coli* K12 strains - which are well-adapted to the laboratory environment, and, unlike wild type strains, have lost their ability to thrive in the intestine. Well-known examples of the *E. coli* K12 strains are K12 Wild type, W3110, MG1655, M182, MC1000, MC1060, MC1061, MC4100, JM101, NZN111 and AA200. Hence, preferably the present invention specifically relates to an *E. coli* K12 strain, more preferably an *E. coli* MG1655 strain. Said bacterium belonging to the phylum Firmicutes belongs preferably to the Bacilli, preferably from the species Bacillus, such as *Bacillus subtilis* or, *B. amyloliquefaciens.* Said bacterium belonging to the phylum Actinobacteria, preferably belonging to the family of the Corynebacteriaceae, with members *Corynebacterium glutamicum* or *C*. *afermentans,* or belonging to the family of the Streptomycetaceae with members *Streptomyces griseus* or *S. fradiae.*

Throughout the application and claims, unless specifically stated otherwise, a yeast cell preferably belongs to the phylum of the Ascomycota or the phylum of the Basidiomycota or the phylum of the Deuteromycota or the phylum of the Zygomycetes. Said yeast cell belongs preferably to the genus *Saccharomyces* (with members like e.g. *Saccharomyces cerevisiae, S. bayanus, S. boulardii), Pichia* (with members like e.g. *Pichia postoris, P. anomala, P. kluyveri), Komagataella, Hansunella, Kluyveromyces* (with members like e.g. *Kluyveromyces lactis, K. marxianus, K. thermotolerans), Yarrowia* (like e.g. *Yarrowia lipolytica), Eremothecium, Zygosaccharomyces, Starmerella* (like e.g. *Starmerella bombicolo) or Debaromyces.* Said yeast cell is more preferably selected from *Pichia pastoris, Yarrowia lipolitica, Saccharomyces cerevisiae and Kluyveromyces lactis.*

Throughout the application and claims, unless specifically stated otherwise, a fungus preferably belongs to the genus *Rhizopus, Dictyostelium, Penicillium, Mucor* or *Aspergillus.*

Throughout the application and claims, unless specifically stated otherwise, a plant cell includes cells of flowering and non-flowering plants, as well as algal cells, for example Chlamydomonas, Chlorella, etc. Preferably, said plant cell is a tobacco, alfalfa, rice, cotton, rapeseed, tomato, corn, maize or soybean cell. Throughout the application and claims, unless specifically stated otherwise, an animal cell is preferably derived from non-human mammals (e.g. cattle, buffalo, pig, sheep, mouse, rat), birds (e.g. chicken, duck, ostrich, turkey, pheasant), fish (e.g. swordfish, salmon, tuna, sea bass, trout, catfish), invertebrates (e.g. lobster, crab, shrimp, clams, oyster, mussel, sea urchin), reptiles (e.g. snake, alligator, turtle), amphibians (e.g. frogs) or insects (e.g. fly, nematode) or is a genetically modified cell line derived from human cells excluding embryonic stem cells. Both human and non-human mammalian cells are preferably chosen from the list consisting of an epithelial cell like e.g. a mammary epithelial cell, an embryonic kidney cell (e.g. HEK293 or HEK 293T cell), a fibroblast cell, a COS cell, a Chinese hamster ovary (CHO) cell, a murine myeloma cell like e.g. an N20, SP2/0 or YB2/0 cell, an NIH-3T3 cell, a non-mammary adult stem cell or derivatives thereof such as described in WO21067641.

Throughout the application and claims, unless specifically stated otherwise, an insect cell is preferably derived from *Spodoptera frugiperda* like e.g. Sf9 or Sf21 cells, *Bombyx mori, Mamestra brassicae, Trichoplusia ni* like e.g. BTI-TN-5B1-4 cells or *Drosophila melanogaster* like e.g. Drosophila S2 cells. Throughout the application and claims, unless specifically stated otherwise, a protozoan cell is preferably *a Leishmania tarentolae* cell.

In another preferred embodiment, said cell used in a biotechnological production process according to the invention is a single cell. It is further preferred that said cell used in a biotechnological production process according to the invention is an isolated cell.

In a more preferred embodiment of the present invention, when said method is an enzymatic process at least one enzyme used in the enzymatic process is immobilized. Preferably said enzyme is immobilised on a carrier or immobilised by entrapping, adsorption or covalent binding. Said enzyme is immobilized by well-known technologies such as by entrapping the enzymes ('Entrapped immobilized enzymes') wherein said enzyme is entrapped in for instance collagen, gelatin, cellulose triacetate, polyacrylamide, and/or j-carrageenan, by binding (Bound immobilized enzymes), by adsorption ('Adsorbed bound immobilized enzymes') wherein said enzyme is adsorbed to polysaccharide derivatives, synthetic polymers, and porous glass, by covalent binding ('Covalently bound immobilized enzymes') wherein said enzyme is bound with glutaraldehyde, bisdiazobenzidine, and hexamethylene, diisocyanate (see e.g. Rafiq Khan M, Bulletin of the National Research Centre 45, 207 (2021)).

In an alternative or more preferred embodiment of the present invention, when said method is an enzymatic process as described herein at least one reaction in the enzymatic process is performed between 50°C and 80°C, preferably between 55°C and 70°C.

In a more preferred embodiment of the present invention, when said method is a fermentation process or a bioconversion, the enzymatic conversions in said fermentation process or bioconversion are performed by at least one enzyme produced by a cell synthesizing said enzyme.

In an alternative more preferred embodiment of the present invention, when said method is a fermentation process, said lactose is produced by a cell genetically engineered to produce all enzymes needed for production of lactose.

In a preferred embodiment of the present invention, the fermentation process or bioconversion process is performed at a temperature between about 25°C and about 50°C, preferably between about 25°C and about 40°C.

In a preferred embodiment of the present invention the carbon source used for conversion by the synthase into lactose is chosen from the list of sucrose or combination of sucrose and glucose.

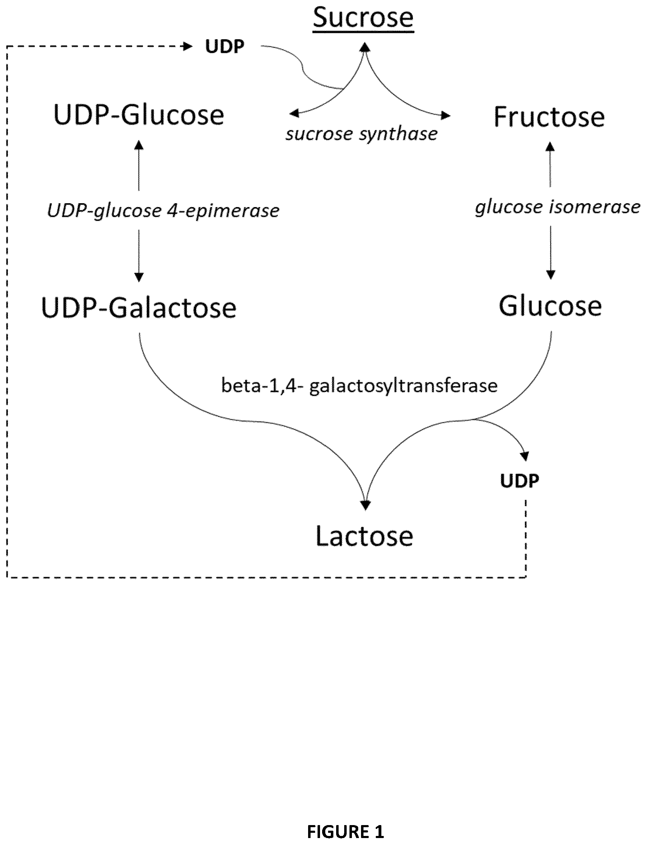

In a preferred embodiment of the present invention, the method uses a synthase which is a sucrose synthase and the method uses sucrose to start the conversion from. Said sucrose is converted with said sucrose synthase into UDP-glucose and fructose. The formed UDP-glucose is converted into UDP-galactose. Said fructose is converted into glucose and said UDP-galactose and said glucose are then converted into lactose. An exemplary embodiment hereof can be found in figure 1.

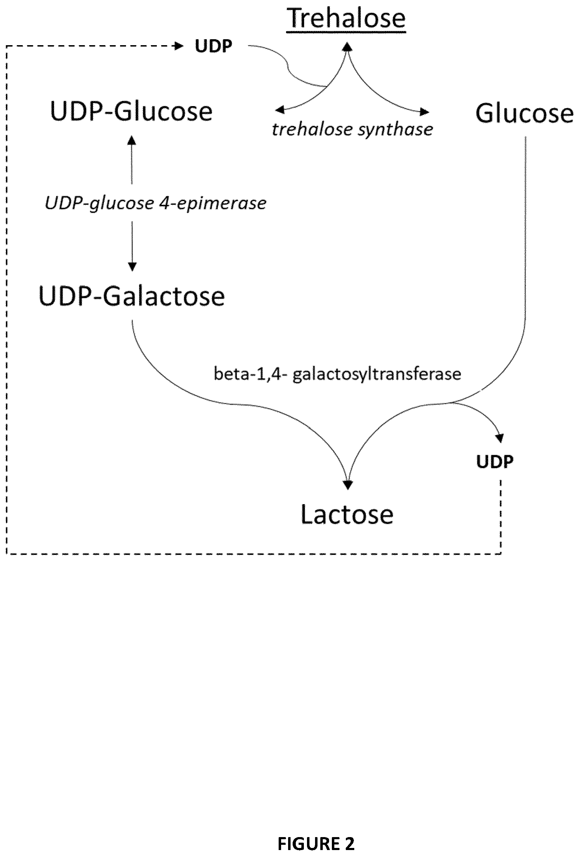

An alternative method, not according to the present invention, can use a synthase which is a trehalose synthase and the method then uses trehalose to start the conversion from. Said trehalose is converted with said trehalose synthase into UDP-glucose and glucose. The formed UDP-glucose is converted into UDP-galactose and said UDP-galactose is then together with glucose converted into lactose. This is described in figure 2.

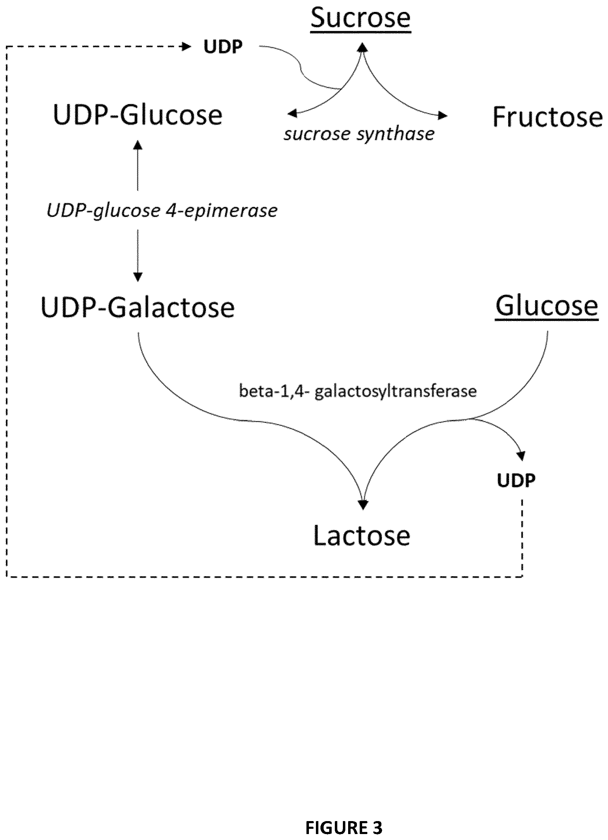

In another alternative preferred embodiment of the present invention, the method uses a synthase which is a sucrose synthase and the method uses sucrose and glucose to start the conversion from. Said sucrose is converted with said sucrose synthase into UDP-glucose and fructose. The formed UDP-glucose is converted into UDP-galactose. No need to convert the fructose into glucose, because glucose is also present as carbon source. Said UDP-galactose and said glucose are then converted into lactose. An exemplary embodiment hereof can be found in figure 3 wherein the carbon sources are underlined.

In a further preferred embodiment of the present invention, the method is an enzymatic production of lactose and uses i) sucrose, or ii) sucrose and glucose as a raw material or carbon source. Lactose is then produced by using i) sucrose, or ii) sucrose and glucose as a raw material or carbon source, and the reaction thereof is catalysed by a plurality of enzyme combinations. In one preferred embodiment all enzymatic reactions are performed in one pot. In another preferred embodiment, some enzymatic reactions are performed separate from the other reactions in the method.

According to a preferred embodiment of the present invention, the method as described herein further comprises a step of separating said lactose.

According to a preferred embodiment of the present invention, the method as described herein further comprises a step of purification of said lactose.

According to a preferred embodiment of the present invention, the method as described herein comprises a step of drying or crystallisation of said lactose, preferably said step of drying comprises any one or more of spray drying, lyophilization, evaporation, precipitation, spray freeze drying, freeze spray drying, band drying, belt drying, vacuum band drying, vacuum belt drying, drum drying, roller drying, vacuum drum drying, vacuum roller drying, and agitated thin film drying.

In a second aspect, the present invention provides a method to purify a biotechnologically synthesized lactose wherein said lactose is purified by at least one of the following purification steps: microfiltration, centrifugation, ultrafiltration, nanofiltration, ion exchange, Simulated Moving Bed (SMB), a colour removal, removal of the carbon source, removal of sucrose or glucose by addition of yeast, removal of sucrose by addition of an invertase, and preferably further comprises concentration, drying and/or crystallization as described herein. Preferably said ion exchange step is a cation and/or anion exchange step.

According to a preferred embodiment of the present invention, the lactose purity before purification is < 70%, < 60%, < 50%, < 40%, < 30%, < 20%, < 10% on total solids, and/or the purity at the end of said method comprising purification is higher than > 80% on dry solids, preferably > 85% on dry solids , more preferably > 90% on dry solids, even more preferably > 95% on dry solids, even more preferably > 97% on dry solids, even more preferably > 98% on dry solids, most preferably > 99% on dry solids.

In a further preferred embodiment, purification of an enzymatically synthesized lactose comprises a step of a microfiltration or ultrafiltration to recover the used enzymes.

In an alternative further preferred embodiment, purification of a biotechnologically synthesized lactose comprises a step of centrifugation, microfiltration and/or ultrafiltration to remove biomass.

In a further preferred embodiment, cells obtained from said fermentation or bioconversion upon purification of said fermentation or bioconversion medium are reused in a further fermentation or a bioconversion process.

In a further preferred embodiment of the present invention, the methods as described herein can comprise i) a step of nanofiltration to remove/reduce monosaccharides and/or salts; and/or ii) a step of ion exchange to remove charged materials.

According to the invention as described herein the purified lactose preferably has an ash content below 1% on total solid, more preferably below 0,5% on total solid. According to a further preferred embodiment, the purified lactose has one or more of:
a) Lead content lower than 0,1 mg/kg solid, preferably lower than 0,02 mg/kg solid;
b) Arsenic content lower than 0,2 mg/kg solid, preferably lower than 0,02 mg/kg solid;
c) Cadmium content lower than 0,1 mg/kg solid, preferably lower than 0,01 mg/kg solid; or
d) Mercury content was lower than 0,5 mg/kg solid, preferably lower than 0,1 mg/kg solid.

In another preferred embodiment according to the present invention, the purified lactose has at least one or more of: i) a protein content below 100 mg per kg dry solid, ii) DNA content below 10 ng per gram dry solid and iii) endotoxin content below 10000 EU per gram dry solid.

Based on the description, the embodiments and the examples in the present invention, all other embodiments obtained by those skilled in the art without inventive effort fall within the scope of protection of the present invention.

In summary, the process for producing lactose of the present invention is completely different from the known processes for producing lactose, and the final product lactose can be used in industries such as food products.

Further advantages follow from the specific embodiments and the examples. It goes without saying that the abovementioned features and the features which are still to be explained below can be used not only in the respectively specified combinations, but also in other combinations or on their own, without departing from the scope of the present invention.

### Examples

### Example 1: Materials and Methods

### Analytical analysis

Standards such as but not limited to sucrose, lactose, glucose, fructose, UDP-glucose, UDP-galactose were purchased from Sigma, Carbosynth (UK), Elicityl (France) and IsoSep (Sweden). Other compounds were analyzed with in-house made standards.

The carbohydrates were analyzed on a Waters Acquity H-class UPLC with Evaporative Light Scattering Detector (ELSD) or a Refractive Index (RI) detection. A volume of 0.7 µL sample was injected on a Waters Acquity UPLC BEH Amide column (2.1 x 100 mm;130 Å;1.7 µm) column with an Acquity UPLC BEH Amide VanGuard column, 130 Å, 2.1x 5 mm. The column temperature was 50 °C. The mobile phase consisted of a ¼ water and ¾ acetonitrile solution to which 0.2 % triethylamine was added. The method was isocratic with a flow of 0.130 mL/min. The ELS detector had a drift tube temperature of 50 °C and the N2 gas pressure was 50 psi, the gain 200 and the data rate 10 pps. The temperature of the RI detector was set at 35 °C.

### Ash content

The ash content is a measure of the total amount of minerals present within a food or ingredients such as oligosaccharides, whereas the mineral content is a measure of the amount of specific inorganic components present within a food, such as Ca, Na, K, Mg, phosphate, sulphate and Cl. Determination of the ash and mineral content of foods or oligosaccharides is important for a number of reasons: Nutritional labeling. The concentration and type of minerals present must often be stipulated on the label of a food or ingredient such as oligosaccharides. The quality of many foods depends on the concentration and type of minerals they contain, including their taste, appearance, texture and stability. Microbiological stability. High mineral contents are sometimes used to retard the growth of certain microorganisms. Nutrition. Some minerals are essential to a healthy diet (e.g., calcium, phosphorous, potassium and sodium) whereas others can be toxic (e.g., lead, mercury, cadmium and aluminium). Processing. It is often important to know the mineral content of foods/products during processing because this affects the physicochemical properties of foods or ingredient such as oligosaccharides.

Ash is the inorganic residue remaining after the water and organic matter have been removed by heating in the presence of oxidizing agents, which provides a measure of the total amount of minerals within a food. Analytical techniques for providing information about the total mineral content are based on the fact that the minerals (the analyte) can be distinguished from all the other components (the matrix) within a food or ingredient in some measurable way. The most widely used methods are based on the fact that minerals are not destroyed by heating, and that they have a low volatility compared to other food components. The three main types of analytical procedure used to determine the ash content of foods are based on this principle: *dry* ashing, *wet* ashing and *low temperature plasma dry* ashing. The method chosen for a particular analysis depends on the reason for carrying out the analysis, the type of food or ingredient analyzed and the equipment available. Ashing may also be used as the first step in preparing samples for analysis of specific minerals, by atomic spectroscopy or the various traditional methods described below.

For the sample preparation a sample whose composition represents that of the ingredient is selected to ensure that its composition does not change significantly prior to analysis. For instance a dry oligosaccharide sample is generally hygroscopic and the selected sample should be kept under dry conditions avoiding the absorption of water. Typically, samples of 1-10g are used in the analysis of ash content. Solid ingredients are finely ground and then carefully mixed to facilitate the choice of a representative sample. Before carrying out an ash analysis, samples that are high in moisture or in solution are generally dried to prevent spattering during ashing. Other possible problems include contamination of samples by minerals in grinders, glassware or crucibles which come into contact with the sample during the analysis. For the same reason, deionized water is used when preparing samples and the same is used in the blank sample.

Dry ashing procedures use a high temperature muffle furnace capable of maintaining temperatures of between 500 and 600 °C. Water and other volatile materials are vaporized and organic substances are burned in the presence of the oxygen in air to CO₂, H₂O and N₂. Most minerals are converted to oxides, sulfates, phosphates, chlorides or silicates. Although most minerals have fairly low volatility at these high temperatures, some are volatile and may be partially lost, e.g., iron, lead and mercury, for these minerals ICP-MS analysis of the product is more appropriate for quantification.

The food sample is weighed before and after ashing to determine the concentration of ash present. The ash content can be expressed on dry basis and is calculated by dividing the mass of the ashed material, ingredient, or food by the mass of the dry material, ingredient, or food before ashing. Multiplied with 100, this gives the percentage of ash in the material, ingredient, or food. In a similar way the wet ash percentage can be determined for liquid products, wherein the mass of the liquid before and after ashing is used instead of the mass of the dry material, ingredient, or food.

### Heavy metal determination

A robust general inductively coupled plasma-mass spectrometry (ICP-MS) based method was used for the detection and quantitation for each of the following elements: arsenic (As), selenium (Se), cadmium (Cd), tin (Sn), lead (Pb), silver (Ag), palladium (Pd), platinum (Pt), mercury (Hg), molybdenum (Mo), sodium (Na), potassium (K), Calcium (Ca), Magnesium (Mg), Iron (Fe), zinc (Zn), manganese (Mn), Phosphorus (P), selenium (Se).

Nitric acid (≥ 65%, Sigma-Aldrich) was used for microwave digestion and standard/sample preparation. All dilutions were done using 18.2 MΩ·cm (Millipore, Bedford, MA, USA) de-ionized water (DIW). About 0.2 g of each oligosaccharide, ingredient, sample were digested in 5 mL of HNO3 using the microwave digestion (CEM, Mars 6) program 15 minutes (min) ramping time and 15 min holding time at 100W and 50°C followed by 15 min ramping time and 20 min holding time at 1800 W and 210°C. The samples were cooled after digestion for 30 minutes. The fully digested samples were then diluted to 50 mL with DIW.

Analyses were carried out using a standard Agilent 7800 ICP-MS, which includes the fourth-generation ORS cell system for effective control of polyatomic interferences using helium collision mode (He mode). The ORS controls polyatomic interferences using He to reduce the transmission of all common matrixbased polyatomic interferences. Smaller, faster analyte ions are separated from larger, slower interference-ions using kinetic energy discrimination (KED). All elements, except Se, were measured in He mode with a flow rate of 5 mL/min. Se was measured in High Energy He (HEHe) mode, using a cell gas flow rate of 10 mL/min. The 7800 ICP-MS was configured with the standard sample introduction system consisting of a MicroMist glass concentric nebulizer, quartz spray chamber, quartz torch with 2.5 mm i.d. injector, and nickel interface cones. The ICP-MS operating conditions are: 1550 W RF power, 8mm sampling depth, 1.16 l/min nebulizing gas, autotuned lens tuning, 5 or 10 ml/min helium gas flow, 5 V KED.

### Dry matter and moisture content quantification

Sartorius MA150 Infrared Moisture Analyzer is used to determine the dry matter content of the oligosaccharides. 0.5g of oligosaccharide is weighed on an analytical balance and is dried in the infrared moisture analyzer until the weight of the sample is stable. The mass of the dried sample divided by the mass of the sample before drying gives the dry matter content (in percent) of the oligosaccharides or sample including oligosaccharides. In a similar way a liquid sample is weighed, however, the amount of liquid weighed is adapted to the expected amount of dry matter in the liquid, so the mass of the dry matter is properly measurable on an analytical balance.

A moisture analyser measures the dry matter, but not the water content. Karl Fisher titration is used to determine the amount of water present in a powder, ingredient of food. The KF titration is carried out with a Karl Fischer titrator DL31 from Mettler Toledo using the two-component technique with Hydra-Point Solvent G and Hydra-Point titrant (5 mg H₂O/ml), both purchased from J.T. Baker (Deventer, Holland). The polarising current for bipotentiometric end-point determination was 20 microA and the stop voltage 100 mV. The end-point criterion was the drift stabilisation (15 micro gram H₂O min⁻¹) or maximum titration time (10 min).

The moisture content (MC) of sample was calculated using the following equation:
MC = V_KF W_eq 100/ W_sample ; where V_KF is the consumption of titrant in mL, W_eq the titer of titrant in mg H₂O/mL and W_sample the weight of sample in mg.

### Protein quantification

For protein quantification a method is used that is compatible with reducing agents, such as reducing sugars or oligosaccharides with a reducing end. To this end, a Bradford assay (Thermo Scientific, Pierce) was used with a linear range between 1 and 1500 µg/ml. The assay was calibrated with a standard curve of BSA. The protein content of dried oligosaccharide products was quantified by dissolving a pre-weighed quantity in 18.2 MΩ·cm (Millipore, Bedford, MA, USA) de-ionized water (DIW) up to a quantity of 50% (m/v). The amount of protein is measured at 595 nm and converted to concentration with the calibration curve based on BSA.

### DNA quantification

Production host specific DNA residue is quantified by RT-qPCR, for which specific primers on the host are designed so that residual DNA of the production host is amplified. The RT-qPCR was performed according to the standard protocol of a kit obtained from Sigma and was based on SYBR Green detection.

Total DNA is measured by means of a Threshold assay (Molecular Devices), based on an immunoassay allowing to measure as low as 2 pg of DNA in a sample in solution. Double stranded DNA is measured by means of SpectraMax^{®} Quant^{™} AccuBlue^{™} Pico dsDNA Assay Kit (Molecular Devices) having a linear range between 5 pg and 3 ng of dsDNA.

### Endotoxin measurement

Endotoxin in the liquid was measured by means of a LAL test.

### A. Escherichia coli

### Media, cultivation and cell lysis

The Luria Broth (LB) medium consisted of 1% tryptone peptone (Difco, Erembodegem, Belgium), 0.5% yeast extract (Difco) and 0.5% sodium chloride (VWR. Leuven, Belgium). The minimal medium used in cultivation experiments in 96-well plates or in shake flasks contained 2.00 g/L NH₄Cl, 5.00 g/L (NH₄)₂SO₄, 2.993 g/L KH₂PO₄, 7.315 g/L K₂HPO₄, 8.372 g/L MOPS, 0.5 g/L NaCl, 0.5 g/L MgSO₄.7H₂O, 30 g/L sucrose or 30 g/L glycerol, 1 ml/L vitamin solution, 100 µl/L molybdate solution, and 1 mL/L selenium solution. The minimal medium was set to a pH of 7 with 1M KOH. Vitamin solution consisted of 3.6 g/L FeCl₂.₄H₂O, 5.0 g/L CaCl₂.2H₂O, 1.3 g/L MnCl₂.2H₂O, 0.38 g/L CuCl₂.2H₂O, 0.5 g/L CoCl₂.6H₂O, 0.94 g/L ZnCl₂, 0.0311 g/L H₃BO₄, 0.4 g/L Na₂EDTA.2H₂O and 1.01 g/L thiamine.HCl. The molybdate solution contained 0.967 g/L NaMoO₄.2H₂O. The selenium solution contained 42 g/L SeO2. The minimal medium for fermentations contained 6.75 g/L NH₄Cl, 1.25 g/L (NH₄)₂SO₄, 2.93 g/L KH₂PO₄ and 7.31 g/L KH₂PO₄, 0.5 g/L NaCl, 0.5 g/L MgSO₄.7H₂O, 30 g/L sucrose or 30 g/L glycerol, 1 mL/L vitamin solution, 100 µL/L molybdate solution, and 1 mL/L selenium solution with the same composition as described above. Complex medium was sterilized by autoclaving (121°C, 21 min) and minimal medium by filtration (0.22 µm Sartorius). When necessary, the medium was made selective by adding an antibiotic: e.g., chloramphenicol (20 mg/L), carbenicillin (100 mg/L), spectinomycin (40 mg/L) and/or kanamycin (50 mg/L). When necessary, inducer was added.

For fermentative production, a preculture of 96-well microtiter plate experiments was started from a cryovial, in 150 µL LB and was incubated overnight at 37 °C on an orbital shaker at 800 rpm. This culture was used as inoculum for a 96-well square microtiter plate, with 400 µL minimal medium by diluting 400x. These final 96-well culture plates were then incubated at 37°C on an orbital shaker at 800 rpm for 72h, or shorter, or longer. To measure sugar concentrations at the end of the cultivation experiment whole broth samples were taken from each well by boiling the culture broth for 15 min at 60°C before spinning down the cells (= average of intra- and extracellular sugar concentrations).

A preculture for the bioreactor was started from an entire 1 mL cryovial of a certain strain, inoculated in 250 mL or 500 mL minimal medium in a 1 L or 2.5 L shake flask and incubated for 24 h at 37°C on an orbital shaker at 200 rpm. A 5 L bioreactor was then inoculated (250 mL inoculum in 2 L batch medium); the process was controlled by MFCS control software (Sartorius Stedim Biotech, Melsungen, Germany). Culturing condition were set to 37 °C, and maximal stirring; pressure gas flow rates were dependent on the strain and bioreactor. The pH was controlled at 6.8 using 0.5 M H₂SO₄ and 20% NH₄OH. The exhaust gas was cooled. 10% solution of silicone antifoaming agent was added when foaming raised during the fermentation.

For heterologous expression of enzyme, a preculture of 96-well microtiter plate experiments was started from a cryovial, in 175 µL LB and was incubated overnight at 37°C on an orbital shaker at 800 rpm. This culture was used as inoculum for a 96-well deep well microtiter plate, with 1 mL LB medium by diluting 100x. These final 96-well culture plates were then incubated at 37°C on an orbital shaker at 800 rpm, induced if necessary, and further grown at 16-37°C for 5-24h. After expression, DW plates were centrifuged for 30 min at 4200 rpm and 4°C, the supernatant was discarded and the pellets were frozen at -80°C for at least one hour. Pellets were subsequently resuspended in buffer containing 1 mg/ml of lysozyme, 10 U/mL DNase and 1 mM protease inhibitor and incubated for 30 min at 37°C. Cell lysates were optionally cleared by centrifuging the plates (30 min at 4200 rpm and 4°C) and transferring the supernatant to new plates.

A preculture of a shake flask experiment was started from a cryovial in 5 mL LB medium and was incubated overnight at 37°C on an orbital shaker at 200 rpm. This culture was used as inoculum for a shake flask with 50-250 mL LB medium by diluting 50x. These final cultures were then incubated at 37°C on an orbital shaker at 200 rpm, induced if necessary, and further grown at 16-37°C for 5-24h, or longer or shorter. After expression, cells were harvest by centrifuging for 30 min at 4200 rpm and 4°C. The supernatant was discarded and the pellets were frozen at -20°C for at least one hour. Pellets were resuspended and lysed by sonication. Cell lysates were optionally cleared by centrifuging (30 min at 4200 rpm and 4°C) and the supernatant was transferred to a new recipient.

A preculture for the bioreactor was started from an entire 1 mL cryovial of a certain strain, inoculated in 250 mL or 500 mL LB medium in a 1 L or 2.5 L shake flask and incubated for 24 h at 37°C on an orbital shaker at 200 rpm. A 5 L bioreactor was then inoculated (250 mL inoculum in 2 L batch medium); the process was controlled by MFCS control software (Sartorius Stedim Biotech, Melsungen, Germany).

Culturing condition were set to 25-37°C, and maximal stirring; pressure gas flow rates were dependent on the strain and bioreactor. The pH was controlled at 6.8 using 0.5 M H₂SO₄ and 20% NH₄OH. The exhaust gas was cooled. 10% solution of silicone antifoaming agent was added when foaming raised during the fermentation. A soft release of the product was established by physically disrupting cells by means of sonication. Other commonly used methods known in the art are methods such as, freeze thawing and/or shear stress through mixing, a homogenizer and/or French press.

Cells for whole cell biotransformation were obtained by centrifuging overnight cultures (produced as described above) for 30 min at 4200 rpm and 4°C. The obtained pellets were subsequently resuspended in 0.9% NaCl solution and transferred to a 50 mL tube and centrifuged for 30 min at 4200 rpm and 4°C. The supernatant was removed and 1000 mg of each pellet was taken and resuspended in 2 mL of 0.9% NaCl (cell concentration is 0.5 g/mL). Optionally, the cells could be permeabilized (e.g. xylene).

### Strains and mutations

*Escherichia coli* K12 MG1655 [λ-, F-, rph-1] was obtained from the Coli Genetic Stock Center (US), CGSC Strain#: 7740, in March 2007 and *Escherichia coli* NiCo21 (DE3) from New England Biolabs, in November 2017. Gene disruptions, gene introductions and gene replacements were performed using the technique published by Datsenko and Wanner (PNAS 97 (2000), 6640-6645). All constitutive promoters, UTRs and terminator sequences originated from the libraries described by Cambray et al. (Nucleic Acids Res. 2013, 41(9), 5139-5148), Dunn et al. (Nucleic Acids Res. 1980, 8, 2119-2132), Edens et al. (Nucleic Acids Res. 1975, 2, 1811-1820), Kim and Lee (FEBS Letters 1997, 407, 353-356) and Mutalik et al. (Nat. Methods 2013, No. 10, 354-360). Genes were ordered synthetically at Twist Bioscience (twistbioscience.com) or IDT (eu.idtdna.com) and the codon usage was adapted using the tools of the supplier. All strains were stored in cryovials at -80°C (overnight LB culture mixed in a 1:1 ratio with 70% glycerol).

*E. coli* K12 MG1655 or NiCo21(DE3) was modified with a knock-out of the *E. coli* gene coding for beta galactosidase, *e.g. lacZ,* in case of lactose production through whole cell biotransformation, cell extract(s), enzymatic synthesis with (partially) purified enzymes or fermentative production. Knock out of *lacZ* might not be a requirement in case of enzymatic synthesis of lactose with purified enzymes. For fermentative production the strain is further modified with an appropriate sugar importer, e.g. cscB for sucrose. The strain could additionally be modified by for instance knocking out proteases (e.g. lon, OmpT), nucleotide sugar degrading enzymes (e.g. ushA) or glucose consuming enzymes (e.g. glk). Optionally, the strain could also be modified by overexpressing an exporter (e.g. setA) to excrete the lactose from the cell.

### B. Saccharomyces cerevisiae

### Media, cultivation and cell lysis

Strains were grown on Synthetic Defined yeast medium with Complete Supplement Mixture (SD CSM) or CSM drop-out (SD CSM-Ura, SD CSM-Trp, SD CSM-His) containing 6.7 g/L Yeast Nitrogen Base without amino acids (YNB w/o AA, Difco), 20 g/L agar (Difco) (solid cultures), 22 g/L sucrose monohydrate or glucose, or fructose and 0.79 g/L CSM or 0.77 g/L CSM-Ura, 0.77 g/L CSM-Trp, or 0.77 g/L CSM-His (MP Biomedicals). In general, yeast strains were initially grown on SD CSM plates to obtain single colonies. These plates were grown for 2-3 days at 30°C. Starting from a single colony, a preculture was grown over night in 5 mL at 30°C, shaking at 200 rpm. Subsequent 125 mL shake flask experiments were inoculated with 2% of this preculture, in 25 mL media. These shake flasks were incubated at 30°C with an orbital shaking of 200 rpm. A soft release of the product was established by physically disrupting cells by means of sonication. Other commonly used methods known in the art are methods such as, freeze thawing and/or shear stress through mixing, a homogenizer and/or French press.

### Strains, plasmids and mutations

*S. cerevisiae* BY4742 created by Brachmann et al. (Yeast (1998) 14:115-32) was used, available in the Euroscarf culture collection. All mutant strains were created by homologous recombination or plasmid transformation using the method of Gietz (Yeast 11:355-360, 1995). Genes were expressed using synthetic constitutive promoters, as described by e.g., Blazeck (Biotechnology and Bioengineering, Vol. 109, No. 11, 2012), Redden and Alper (Nat. Commun. 2015, 6, 7810), Liu et al. (Microb. Cell Fact. 2020, 19, 38), Xu et al. (Microb. Cell Fact.2021, 20, 148) and Lee et al. (ACS Synth. Biol. 2015, 4(9), 975-986).

### C. Bacillus subtilis

### Media, cultivation and cell lysis

Two media are used to cultivate *B. subtilis:* i.e., a complex medium like a rich Luria Broth (LB) and a minimal medium for shake flask cultures. The LB medium consisted of 1% tryptone peptone (Difco), 0.5% yeast extract (Difco) and 0.5% sodium chloride (VWR). Luria Broth agar (LBA) plates consisted of the LB media, with 12 g/L agar (Difco) added. The minimal medium contained 2.00 g/L (NH₄)₂SO₄, 7.5 g/L KH₂PO₄, 17.5 g/L K₂HPO₄, 1.25 g/L Na-citrate, 0.25 g/L MgSO₄.7H₂O, 0.05 g/L tryptophan, from 10 up to 30 g/L glucose (or another carbon source including but not limited to fructose, maltose, sucrose, glycerol and maltotriose), 10 mL/L trace element mix and 10 mL/L Fe-citrate solution. The medium was set to a pH of 7 with 1 M KOH. The trace element mix consisted of 0.735 g/L CaCl₂.2H₂O, 0.1 g/L MnCl₂.2H₂O, 0.033 g/L CuCl₂.2H₂O, 0.06 g/L CoCl₂.6H₂O, 0.17 g/L ZnCl₂, 0.0311 g/L H₃BO₄, 0.4 g/L Na₂EDTA.2H₂O and 0.06 g/L Na₂MoO₄. The Fe-citrate solution contained 0.135 g/L FeCl₃.6H₂O, 1 g/L Na-citrate (Hoch 1973 PMC1212887). Complex medium, e.g., LB, was sterilized by autoclaving (121°C, 21 min) and minimal medium by filtration (0.22 µm Sartorius). When necessary, the medium was made selective by adding an antibiotic. *B. subtilis* strains were initially grown on LB agar to obtain single colonies. These plates were grown over night at 37°C. Starting from a single colony, a preculture was grown over night in 5 mL at 37°C, shaking at 200 rpm. Subsequent 125 mL shake flask experiments were inoculated with 2% of this preculture, in 25 mL media. These shake flasks were incubated at 37°C with an orbital shaking of 200 rpm for 72h, or shorter or longer. At the end of the cultivation experiment samples were taken to measure the supernatant concentration (extracellular sugar concentrations, after 5 min. spinning down the cells), or by boiling the culture broth for 15 min at 90°C or for 60 min at 60°C before spinning down the cells (= whole broth concentration, i.e., intra- and extracellular sugar concentrations). A soft release of the product was established by physically disrupting cells by means of sonication. Other commonly used methods known in the art are methods such as, freeze thawing and/or shear stress through mixing, a homogenizer and/or French press.

### Strains, plasmids and mutations

*B. subtilis* 168 is used as available at the Bacillus Genetic Stock Center (Ohio, USA). Plasmids for gene deletion via Cre/lox are constructed as described by Yan et al. (Appl & Environm microbial, Sept 2008, p5556-5562). Gene disruption is done via homologous recombination with linear DNA and transformation via the electroporation as described by Xue et al. (J. microb. Meth. 34 (1999) 183-191). The method of gene knockouts is described by Liu et al. (Metab. Engine. 24 (2014) 61-69). Integrative vectors as described by Popp et al. (Sci. Rep., 2017, 7, 15158) are used as expression vector and could be further used for genomic integrations if necessary. A suitable promoter for expression can be derived from the part repository (iGem): sequence id: BBa_K143012, BBa_K823000, BBa_K823002 or BBa_K823003. Cloning can be performed using Gibson Assembly, Golden Gate assembly, Cliva assembly, LCR or restriction ligation.

### D. Corynebacterium glutamicum

### Media, cultivation and cell lysis

Two different media are used, namely complex medium like e.g., a rich tryptone-yeast extract (TY) medium, and a minimal medium for shake flask (MMsf). The minimal medium uses a 1000x stock trace element mix. Trace element mix consisted of 10 g/L CaCl₂, 10 g/L FeSO₄.7H₂O, 10 g/L MnSO₄.H₂O, 1 g/L ZnSO₄.7H₂O, 0.2 g/L CuSO₄, 0.02 g/L NiCl₂.6H₂O, 0.2 g/L biotin (pH 7) and 0.03 g/L protocatechuic acid. The minimal medium for the shake flasks (MMsf) experiments contained 20 g/L (NH₄)₂SO₄, 5 g/L urea, 1 g/L KH₂PO₄, 1 g/L K₂HPO₄, 0.25 g/L MgSO₄.7H₂O, 42 g/L MOPS, from 10 up to 30 g/L glucose or another carbon source including but not limited to fructose, maltose, sucrose, glycerol and maltotriose when specified in the examples and 1 ml/L trace element mix. The TY medium consisted of 1.6% tryptone (Difco, Erembodegem, Belgium), 1% yeast extract (Difco) and 0.5% sodium chloride (VWR. Leuven, Belgium). TY agar (TYA) plates consisted of the TY media, with 12 g/L agar (Difco, Erembodegem, Belgium) added. Complex medium, e.g., TY, was sterilized by autoclaving (121°C, 21 min) and minimal medium by filtration (0.22 µm Sartorius). When necessary, the medium was made selective by adding an antibiotic.

A preculture was started from a cryovial or a single colony from a TY plate, in 6 mL TY and was incubated overnight at 37 °C on an orbital shaker at 200 rpm. Subsequent 125 mL shake flask experiments were inoculated with 2% of this preculture, in 25 mL MMsf medium. These shake flasks were incubated at 37°C with an orbital shaking of 200 rpm for 72h, or shorter or longer. At the end of the cultivation experiment samples were taken to measure the supernatant concentration (extracellular sugar concentrations, after 5 min. spinning down the cells), or by boiling the culture broth for 15 min at 90°C or for 60 min at 60°C before spinning down the cells (= whole broth concentration, i.e., intra- and extracellular sugar concentrations). A soft release of the product was established by physically disrupting cells by means of sonication. Other commonly used methods known in the art are methods such as, freeze thawing and/or shear stress through mixing, a homogenizer and/or French press.

### Strains and mutations

*Corynebacterium glutamicum* was used as available at the American Type Culture Collection (ATCC 13032). Integrative plasmid vectors were made using the Cre/loxP technique as described by Suzuki et al. (Appl. Microbiol. Biotechnol., 2005 Apr, 67(2):225-33) and temperature-sensitive shuttle vectors as described by Okibe et al. (Journal of Microbiological Methods 85, 2011, 155-163) are constructed for gene deletions, mutations and insertions. Suitable promoters for (heterologous) gene expression can be derived from Yim et al. (Biotechnol. Bioeng., 2013 Nov, 110(11):2959-69). Cloning can be performed using Gibson Assembly, Golden Gate assembly, Cliva assembly, LCR or restriction ligation.

### E. Optical density

Cell density of the cultures was frequently monitored by measuring optical density at 600 nm (Implen Nanophotometer NP80, Westburg, Belgium or with a Spark 10M microplate reader, Tecan, Switzerland). The maximum growth speed (mumax) was calculated based on the observed optical densities at 600nm using the R package grofit.

### F. Growth rate/speed measurement

The maximal growth rate (µMax) was calculated based on the observed optical densities at 600 nm using the R package grofit.

### G. Heterologous and homologous expression

Genes that needed to be expressed, be it from a plasmid or from the genome were synthetically synthetized with one of the following companies: IDT or Twist Bioscience. Proteins described in present disclosure are summarized in Table 1. Unless stated otherwise, the UniProt IDs of the proteins described correspond to their sequence version 01 as present in the UniProt Database version release 2023_02 of 03 May 2023. Expression could be further facilitated by optimizing the codon usage to the codon usage of the expression host. Genes were optimized using the tools of the supplier.

**Table 1 Overview of genes and their UniProt ID used to describe the present invention**

| **Enzyme** | **UniProt ID** | **Organism** | **Origin** | **Country of origin of digital sequence information** |
|---|---|---|---|---|
| SuSy | P13708 | *Glycine max* | Synthetic | unknown |
| SuSy | P49040 | *Arabidopsis thaliana* | Synthetic | USA |
| SuSy | Q9ZEV2 | *Anabaena* sp. PCC 7119 (ATCC no. 29151) | Synthetic | USA |
| SuSy | A0A059ZV61 | *Acidithiobacillus caldus* ATCC 51756 | Synthetic | UK |
| GalE | P09147 | *Escherichia coli* K-12 MG1655 | Synthetic | USA |
| GalE | E8MF10 | *Bifidobacterium longum* subsp. *longum* (strain ATCC 15707 / DSM 20219 / JCM 1217 / NCTC 11818 / E194b) | Synthetic | unknown |
| GalE | A0A0K1E1Q5 | *Pyrobaculum sp. WP30* | Synthetic | USA |
| GalE | Q0P9C3 | *Campylobacter jejuni* subsp. *jejuni* serotype O:2 (strain ATCC 700819 / NCTC 11168) | Synthetic | UK |
| GalT | Q51116 | *Neisseria meningitidis* MC58 | Synthetic | UK |
| XylA | P37031 | *Streptomyces murinus* | Synthetic | unknown |
| XylA | P12070 | *Arthrobacter* sp. (strain NRRL B3728) | Synthetic | unknown |
| XylA | D2DK62 | *Thermoanaerobacter ethanolicus* JW 200 | Synthetic | USA |
| treT | Q7LYW5 (GenPept ID WP_0040687 20) | *Thermococcus bergensis* strain T7324 | Synthetic | Norway |
| treT | O58762 | *Pyrococcus horikoshii* (strain ATCC 700860 / DSM 12428 / JCM 9974 / NBRC 100139 / OT-3) | Synthetic | Japan |
| XylA | P00944 | *Escherichia coli* K-12 MG1655 | Synthetic | USA |
| GalT | D0EAD4 (GenPept ID WP_0217343 30) | *Pasteurella multocida* M1404 | Synthetic | Canada |
| GalE | P26503 | *Sinorhizobium meliloti* (strain 1021) | Synthetic | USA |

### H. Analysis

To determine carbohydrates HPAEC-PAD system (Dionex) is used with a Dionex CarboPac PA1 Guard, 2 × 50 mm and Dionex CarboPac PA1 Separation, 2 × 250 mm is used with gradient of sodium acetate (1M) (A), sodium hydroxide (200mM) (B), water (C) and sodium acetate (25mM) (D), wherein the gradient from 0-10 min is 5% B, 88% C and 7% D, from 10-15 min changing B from 5% to 17% and C from 88% to 76%, from 15-25 min changing B from 17% to 93% and C from 76% to 0%, from 25-28.1 min changing A from 0% to 20% and B from 93% to 73%, from 28.1 min to 32 min maintaining the percentages of A, B C and D, from 32 to 32.1 min changing A from 20 to 0%, B from 73 to 0%, C from 0% to 88%, from 32.1 to 50 min maintaining the percentages of A, B, C, and D.

To determine nucleotide sugars the same system was used, albeit with a different gradient: sodium hydroxide (1 mM) (A) and sodium acetate (1M) in sodium hydroxide (1 mM) (B), wherein the gradient from 0-10 min changing A from 80% to 45% and B from 20% to 55%, from 10-25 min is 45% A and 55% B, from 25-35 changing A from 45% to 20% and B from 55% to 80%, from 35-40 min changing A from 20% to 0% and B from 80% to 100%, from 40-50 min is 0% A and 100% B, from 50-50.001 min changing A from 0% to 80% and B from 100% to 20% and from 50.001-60 min is 80% A and 20% B.

### I. Enzyme purification

Cleared lysates containing N-terminal histidine-tagged enzymes in 50 mM Tris pH 7.0, 250 mM NaCl and 10 mM imidazole were applied on an ÄKTA Pure Protein Purification System, equipped with a Ni-NTA column. Bound protein was washed with five column volumes of wash buffer (50mM Tris, pH 7.0, 250 mM NaCl, 20-50 mM imidazole and eluted with elution buffer (50 mM Tris, pH 7.0, 250 mM NaCl, 250 mM imidazole) and the protein of interest was collected. Buffer exchange was afterwards performed on an Amicon Centrifugal Filter with the appropriate MW cut-off. Other commonly used purification methods known in the art are methods such as salting out, size exclusion chromatography, affinity chromatography, ion-exchange chromatography and/or any combination of the aforementioned methods.

### J. SDS-PAGE and Western Blot

Three microL Laemmli 4x buffer containing 2-mercaptoethanol (1:10) was added to 9 microL of protein sample (final protein concentration of 5 mg/mL for cell extract or 0.05-0.5 mg/mL for purified protein) and heated for 5 min at 95°C. The protein samples were loaded together with a protein ladder and run at 120-220 V. After electrophoresis, gels were either stained (1 h in Coomassie Brilliant Blue R-250) or transferred to a blot membrane for Western Blot. The membrane with transferred protein was blocked for at least 30 minutes with 5% milk powder and 0.2% Tween 20, prior to the application of the primary antibody (anti-His). After 2 hours of incubation, the membrane was washed and HRP-linked secondary antibody was applied for one hour. Finally, the membrane was washed again and the his-tagged proteins were visualised by means of chemiluminescence.

### Example 2: Expression of active sucrose synthase

An *E. coli* NiCo21(DE3) strain as described in Example 1 was transformed with an inducible expression plasmid containing the gene coding for the sucrose synthase (SuSy) from either G. *max* UniProt ID P13708, *A. thaliana* UniProt ID P49040, *Anabaena* sp. UniProt ID Q9ZEV2 or *A. caldus* UniProt ID A0A059ZV61. Expression of the enzymes was performed in a shake flask and cells were harvested and lysed as described in Example 1. Western blot analysis on the cleared cell lysates was performed as described in Example 1 and confirmed the expression of the target enzymes. Activity was verified by incubating 100 mM sucrose, 10 mM UDP and 50% (v/v) SuSy cell extract in 50 mM MOPS buffer (pH 7.0) for 24 h at 37°C and analysing the formation of UDP-glucose and fructose as described in Example 1.

### Example 3: Expression of active UDP-glucose-4-epimerase

An *E. coli* NiCo21(DE3) strain as described in Example 1 was transformed with an inducible expression plasmid containing the gene coding for the UDP-glucose-4-epimerase from either *E. coli* UniProt ID P09147, *B. longum* UniProt ID E8MF10, *P. sp. WP30*UniProt ID A0A0K1E1Q5 or *C.jejuni* UniProt ID Q0P9C3. Expression of the enzymes was performed in a shake flask and cells were harvested and lysed as described in Example 1. Western blot analysis on the cleared cell lysates was performed as described in Example 1 and confirmed the expression of the target enzymes. Activity was verified by incubating 10 mM UDP-glucose and 50% (v/v) GalE cell extract in 50 mM MOPS buffer (pH 6.5) for 24 h at 37°C and analysing the formation of UDP-galactose as described in Example 1.

### Example 4: Expression of active beta 1,4-galactosyltransferase

An *E. coli* NiCo21(DE3) strain as described in Example 1 was transformed with an inducible expression plasmid containing the gene coding for the beta 1,4-galactosyltransferase (GalT) from *N. meningitidis* with UniProt ID Q51116. Expression of the enzyme was performed in a shake flask and cells were harvested and lysed as described in Example 1. Western blot analysis on the cleared cell lysate was performed as described in Example 1 and confirmed the expression of the target enzyme. Activity was verified by incubating 10 mM UDP-galactose, 10 mM glucose, 10 mM MgCl₂ and 50% (v/v) cell extract in 50 mM MOPS buffer (pH 7.0) for 24 h at 37°C and analysing the formation of lactose as described in Example 1.

### Example 5: Expression of active glucose isomerase

An *E. coli* NiCo21(DE3) strain as described in Example 1 was transformed with an inducible expression plasmid containing the gene coding for the glucose isomerase from *S. murinis* UniProt ID P37031, *Arthrobacter* UniProt ID P12070 or *T. ethanolicus* UniProt ID D2DK62. Expression of the enzymes was performed in a shake flask and cells were harvested and lysed as described in Example 1. Western blot analysis on the cleared cell lysates was performed as described in Example 1 and confirmed the expression of the target enzymes. Activity was verified by incubating 100 mM fructose, 10 mM MgCl₂ and 50% (v/v) cell extract in 50 mM MOPS buffer (pH 6.5) for 24 h at 37°C and analysing the formation of glucose as described in Example 1.

### Example 6: Expression of active trehalose synthase

An *E. coli* NiCo21(DE3) strain as described in Example 1 was transformed with an inducible expression plasmid containing the gene coding for the trehalose synthase (treT) from *T. bergensis* UniProt ID Q7LYW5 or *P. horikoshii* with UniProt ID O58762. Expression of the enzyme was performed in a shake flask and cells were harvested and lysed as described in Example 1. Western blot analysis on the cleared cell lysates was performed as described in Example 1 and confirmed the expression of the target enzyme. Activity was verified by incubating 10 mM trehalose, 10 mM UDP, 10 mM MgCl₂ and 50% (v/v) cell extract in 50 mM MOPS buffer (pH 6.5) for 24 h at 37°C and analysing the formation of glucose as described in Example 1.

### Example 7: Activity of sucrose synthase at elevated temperature

An *E. coli* NiCo21(DE3) strain as described in Example 1 was transformed with an inducible expression plasmid containing the gene coding for the sucrose synthase (SuSy) from *A. caldus* with UniProt ID ADA059ZV61. Expression of the enzyme was performed in a shake flask and cells were harvested and lysed as described in Example 1. Activity was verified by incubating 100 mM sucrose, 10 mM UDP and 50% (v/v) SuSy cell extract in 50 mM MOPS buffer (pH 7.0) for 1 h at 60°C and analysing the formation of UDP-glucose and fructose as described in Example 1.

### Example 8: Activity of UDP-glucose-4-epimerase at elevated temperature

An *E. coli* NiCo21(DE3) strain as described in Example 1 was transformed with an inducible expression plasmid containing the gene coding for the UDP-glucose-4-epimerase from *P. sp. WP30* with UniProt ID A0A0K1E1Q5. Expression of the enzyme was performed in a shake flask and cells were harvested and lysed as described in Example 1. Activity was verified by incubating 10 mM UDP-glucose and 50% (v/v) GalE cell extract in 50 mM MOPS buffer (pH 6.5) for 1 h at 60°C and analysing the formation of UDP-galactose as described in Example 1.

### Example 9: Activity of glucose isomerase at elevated temperature

An *E. coli* NiCo21(DE3) strain as described in Example 1 was transformed with an inducible expression plasmid containing the gene coding for the glucose isomerase from *T. ethanolicus with* UniProt ID D2DK62. Expression of the enzyme was performed in a shake flask and cells were harvested and lysed as described in Example 1. Activity was verified by incubating 100 mM fructose, 10 mM MgCl₂ and 50% (v/v) cell extract in 50 mM MOPS buffer (pH 6.5) for 1 h at 60°C and analysing the formation of glucose as described in Example 1.

### Example 10: Activity of trehalose synthase at elevated temperature

An *E. coli* NiCo21(DE3) strain as described in Example 1 was transformed with an inducible expression plasmid containing the gene coding for the trehalose synthase (treT) from *T. bergensis* UniProt ID Q7LYW5 or *P. horikoshii* with UniProt ID O58762. Expression of the enzyme was performed in a shake flask and cells were harvested and lysed as described in Example 1. Activity was verified by incubating 10 mM trehalose, 10 mM UDP, 10 mM MgCl₂ and 50% (v/v) cell extract in 50 mM MOPS buffer (pH 6.5) for 1 h at 60°C and analysing the formation of glucose as described in Example 1.

### Example 11: Co-expression sucrose synthase, UDP-glucose-4-epimerase and beta 1,4-galactosyltransferase

An E. coli NiCo21(DE3) strain as described in Example 1 was transformed with an expression plasmid containing a constitutive transcriptional unit for the sucrose synthase (SuSy) from *A. caldus* with UniProt ID ADA059ZV61, a constitutive transcriptional unit for the UDP-glucose-4-epimerase (GalE) from P. *sp. WP30* with UniProt ID A0A0K1E1Q5, and a constitutive transcriptional unit for the beta 1,4-galactosyltransferase (GalT) from *N. meningitidis* with UniProt ID Q51116. Expression of the enzymes was performed in a shake flask and cells were harvested and lysed as described in Example 1. Western blot analysis was performed on the cleared cell lysates as described in Example 1 and confirmed the expression of the target enzymes.

### Example 12: Co-expression sucrose synthase, UDP-glucose-4-epimerase, beta 1,4-galactosyltransferase and glucose isomerase

An *E. coli* NiCo21(DE3) strain as described in Example 1 was transformed with an expression plasmid containing a constitutive transcriptional unit for the sucrose synthase (SuSy) from *A. caldus* with UniProt ID ADA059ZV61, a constitutive transcriptional unit for the UDP-glucose-4-epimerase (GalE) from *P. sp. WP30* with UniProt ID A0A0K1E1Q5, a constitutive transcriptional unit for the beta 1,4-galactosyltransferase (GalT) from *N. meningitidis* with UniProt ID Q51116 and a constitutive transcriptional unit for the glucose isomerase (XylA) from *T. ethanolicus* with UniProt ID D2DK62. Expression of the enzymes was performed in a shake flask and cells were harvested and lysed as described in Example 1. Western blot analysis was performed on the cleared cell lysates as described in Example 1 and confirmed the expression of the target enzymes.

### Example 13: Co-expression of trehalose synthase, UDP-glucose-4-epimerase and beta 1,4-galactosyltransferase

An *E. coli* NiCo21(DE3) strain as described in Example 1 was transformed with an expression plasmid containing a constitutive transcriptional unit for the trehalose synthase (treT) from *T. bergensis* with UniProt ID Q7LYW5, a constitutive transcriptional unit for the UDP-glucose-4-epimerase (GalE) from *P. sp. WP30* with UniProt ID A0A0K1E1Q5and a constitutive transcriptional unit for the beta 1,4-galactosyltransferase (GalT) from *N. meningitidis* with UniProt ID Q51116. Expression of the enzymes was performed in a shake flask and cells were harvested and lysed as described in Example 1. Western blot analysis was performed on the cleared cell lysates as described in Example 1 and confirmed the expression of the target enzymes.

### Example 14: In vitro synthesis of lactose via cell extracts of individually expressed enzymes

Cell lysates from strains expressing a sucrose synthase, as described in Example 2, a UDP-glucose-4-epimerase, as described in Example 3, and a beta 1,4-galactosyltransferase, as described in Example 4, were mixed with substrate in a one-pot multi-enzyme reaction containing 100 mM sucrose, 100 mM glucose, 2 mM UDP, 10 mM MgCl₂ and three times 16.6% (v/v) cell extract in 50 mM MOPS buffer (pH 7.0) and incubated for 24 h at 37°C. The formation of lactose was analysed as described in Example 1.

### Example 15: In vitro synthesis of lactose via cell extracts of individually expressed enzymes

Cell lysates from strains expressing a sucrose synthase, as described in Example 2, a UDP-glucose-4-epimerase, as described in Example 3, a beta 1,4-galactosyltransferase, as described in Example 4, and a glucose isomerase, as described in Example 5, were mixed with substrate in a one-pot multi-enzyme reaction containing 100 mM sucrose, 2 mM UDP, 10 mM MgCl₂ and four times 12.5% (v/v) cell extract in 50 mM MOPS buffer (pH 7.0) and incubated for 24 h at 37°C. The formation of lactose was analysed as described in Example 1.

### Example 16: In vitro synthesis of lactose via cell extracts of individually expressed enzymes

Cell lysates from strains expressing a trehalose synthase, as described in Example 6, a UDP-glucose-4-epimerase, as described in Example 3, and a beta 1,4-galactosyltransferase, as described in Example 4, were mixed with substrate in a one-pot multi-enzyme reaction containing 100 mM trehalose, 2 mM UDP, 10 mM MgCl₂ and three times 16.6% (v/v) cell extract in 50 mM MOPS buffer (pH 7.0) and incubated for 24 h at 37°C. The formation of lactose was analysed as described in Example 1.

### Example 17: In vitro synthesis of lactose via cell extracts of co-expressed enzymes

Cell lysates from strains co-expressing the three enzymes, as described in Example 11, were mixed with substrate in a one-pot multi-enzyme reaction containing 100 mM sucrose, 100 mM glucose, 2 mM UDP, 10 mM MgCl₂ and 50% (v/v) cell extract in 50 mM MOPS buffer (pH 7.0) and incubated for 24 h at 37°C. The formation of lactose was analysed as described in Example 1.

### Example 18: In vitro synthesis of lactose via cell extracts of co-expressed enzymes

Cell lysates from strains co-expressing the four enzymes, as described in Example 12, were mixed with substrate in a one-pot multi-enzyme reaction containing 100 mM sucrose, 2 mM UDP, 10 mM MgCl₂ and 50% (v/v) cell extract in 50 mM MOPS buffer (pH 7.0) and incubated for 24 h at 37°C. The formation of lactose was analysed as described in Example 1.

### Example 19: In vitro synthesis of lactose via cell extracts of co-expressed enzymes

Cell lysates from strains co-expressing the three enzymes, as described in Example 13, were mixed with substrate in a one-pot multi-enzyme reaction containing 100 mM trehalose, 2 mM UDP, 10 mM MgCl₂ and 50% (v/v) cell extract in 50 mM MOPS buffer (pH 7.0) and incubated for 24 h at 37°C. The formation of lactose was analysed as described in Example 1.

### Example 20: In vitro synthesis of lactose via purified enzymes

Cell lysates from strains expressing a sucrose synthase, as described in Example 2, a glucose isomerase, as described in Example 3, a beta 1,4-galactosyltransferase, as described in Example 4, and a glucose isomerase, as described in Example 5, were purified as described in Example 1. The purified enzymes were subsequently mixed in a one-pot multi-enzyme reaction containing 100 mM sucrose, 2 mM UDP, 10 mM MgCl₂ and 0.1 mg/ml purified enzyme in 50 mM MOPS buffer (pH 7.0) and incubated for 24 h at 37°C. The formation of lactose was analysed as described in Example 1.

### Example 21: In vivo synthesis of lactose

An *E. coli* K-12 MG1655 strain modified for growth on sucrose and deficient in the breakdown of lactose, as described in Example 1, was transformed with an expression plasmid containing a constitutive transcriptional unit for the sucrose synthase (SuSy) from either *G*. *max* UniProt ID P13708, *A. thaliana* UniProt ID P49040, *Anabaena* sp. UniProt ID Q9ZEV2 or *A*. *caldus* UniProt ID A0A059ZV61, a constitutive transcriptional unit for the UDP-glucose-4-epimerase (GalE) from either *E. coli* UniProt ID P09147, B. *longum* UniProt ID E8MF10, *P. sp. WP30* UniProt ID A0A0K1E1Q5 or *C. jejuni* UniProt ID Q0P9C3, a constitutive transcriptional unit for the beta 1,4-galactosyltransferase (GalT) from *N. meningitidis* with UniProt ID Q51116and a constitutive transcriptional unit for the glucose isomerase (XylA) from either *S*. *murinis* UniProt ID P37031, *Arthrobacter* UniProt ID P12070 or *T. ethanolicus* UniProt ID D2DK62, as described in Example 6. The novel strains were evaluated in a 96-well plate for production of lactose according to the culture conditions provided in Example 1 in which the strains were cultivated in minimal medium with 30 g/l sucrose. After 72h of incubation, the culture broth was harvested, and the formation of lactose was analysed as described in Example 1.

### Example 22: Whole cell biotransformation synthesis of lactose

An *E. coli* K-12 MG1655 strain as described in Example 1 was transformed with an expression plasmid containing a constitutive transcriptional unit for the sucrose synthase (SuSy) from *A. caldus* with UniProt ID ADA059ZV61, a constitutive transcriptional unit for the UDP-glucose-4-epimerase (GalE) from *P. sp. WP30* with UniProt ID A0A0K1E1Q5, a constitutive transcriptional unit for the beta 1,4-galactosyltransferase (GalT) from *N. meningitidis* with UniProt ID Q51116 and a constitutive transcriptional unit for the glucose isomerase (XylA) from *T. ethanolicus* with UniProt ID D2DK62. The strain was cultivated in a shake flask and cells were harvested and prepared for whole cell biotransformation as described in Example 1. A reaction vessel containing 100 mM sucrose, 50 mM UDP, 100 mM MOPS, 15 mM MgSO₄ and 50 mg/mL cells, pH 7 was incubated for 48 hours at 21 °C and 175 rpm. The formation of lactose was analysed as described in Example 1.

### Example 23: Recovery on the synthesis enzymes

The enzymes may be recovered from the solution by means of ultrafiltration, wherein the molecular cut-off of the ultrafiltration membrane is smaller than the molecular weight of the enzymes (i.e. for sucrose synthase between 80 and 440 kDa, for UPD-glucose-4-epimerase between 30 and 80 kDa, for beta 1,4-galactosyltransferase between 30 and 45 kDa, for glucose isomerase between 40 and 200 kDa and for trehalose synthase between 45 and 200 kDa). The retained enzymes are recycled to the reaction vessel of column, while the filtrate with the residual substrates, byproducts and salts are further purified to form highly purified lactose.

Typical ultrafiltration membranes are PES membranes (Synder), PAN membranes (Synder), PVDF membranes (Synder), SPES membranes or ceramic membranes (Tami) with molecular weight cut-off ranging between 1kDa and 100kDa.

In a preferred production setup, the ultrafiltration recycling of the enzymes is performed in a continuous manner, enabling continuous lactose synthesis.

### Example 24: Removal of protein, cells, other carbohydrates and salts from the lactose solution

After the synthesis of lactose as described in the previous examples, typical impurities are sucrose, glucose, fructose, galactose, UDP-glucose, UDP-galactose, and buffer components (phosphate salts and cations such as magnesium).

These impurities needs to be removed from the solution, yet separating sugars from each other in a solution is not trivial. These sugars have similar properties and molecular weights and are hence not easily separated.

### The use of a selective yeast to purify lactose

A solution containing lactose, sucrose, glucose, fructose, galactose, UDP-glucose, UDP-galactose, and buffer components and the synthesis enzymes was inoculated with baker's yeast (for instance Bruggeman yeast, *Saccharomyces cerevisiae*). This yeast converts sucrose, fructose, galactose, UDP-glucose, UDP-galactose to biomass and ethanol but leaves the lactose in solution.

After this conversion, the yeast was centrifuged off, leaving in the supernatant lactose, the buffer, and the metabolites formed by *Saccharomyces cerevisiae* (e.g. ethanol, acetate, glycerol) and cell debris. The formation of these metabolites and debris poses a new challenge, these need to be removed. An alternative method for the removal of the yeast is flocculation and decantation or microfiltration with pore size 0.1 to 10µm (ceramic, PES, PVDF membranes).

To this end, the cell debris is removed by ultrafiltration resulting in a solution with less than 100 mg protein per kg dry solid, a DNA content below 10 ng per gram dry solid and an endotoxin was below 10000 EU per gram dry solid. The ultrafiltration was followed by a nanofiltration step in which the buffer salts and yeast metabolites are separated from the lactose. For the nanofiltration a NFX membrane (Synder) with a molecular weight cut-off of 300Da is used at 50°C, which has >99% rejection for lactose. The membrane has a salt rejection <40% which allows easy removal of salts and yeast metabolites while preforming diafiltrations with water. The lactose yield after this step is higher than 95%. During the nanofiltration the solution is further concentrated to a solution of >15% dry matter.

An alternative nanofiltration membrane is NFW (Synder, with molecular weight cut-off <600Da). This membrane has a lactose rejection of >98.5%, salt rejection of <20.0%, which allows easy removal of the salts and yeast metabolites in a diafiltration step. The ash content on total solid after diafiltration is reduced to <5% on total solid.

### The use of an invertase to selectively hydrolyse sucrose

A solution containing lactose, sucrose, glucose, fructose, galactose, UDP-glucose, UDP-galactose, and buffer components and the synthesis enzymes was treated with an invertase (Beta-fructofuranosidase, Novozymes, approx. 1 g/l, pH 5, 50°C). The residual sucrose is converted into glucose and fructose during this treatment, while leaving the lactose intact.

After the sucrose inversion, the enzymes are removed by ultrafiltration resulting in a solution with less than 100 mg protein per kg dry solid, a DNA content below 10 ng per gram dry solid and an endotoxin was below 10000 EU per gram dry solid. The ultrafiltration was followed by a nanofiltration step in which the buffer salts and monosaccharides are separated from the lactose. For the nanofiltration a DL series membrane (Suez) with a molecular weight cut-off of 300Da is used at 50°C, which has >99% rejection for lactose but a low rejection for monosaccharides (5-10%). By diafiltration the monosaccharides and the salts are removed in this step. During the nanofiltration the solution is further concentrated to a solution of >15% dry matter. The ash content on total solid after diafiltration is reduced to <5% on total solid.

### Example 25: Removal of protein, cells, other carbohydrates and salts from the lactose solution

After the synthesis of lactose as described in the previous examples, typical impurities are trehalose glucose, galactose, UDP-glucose, UDP-galactose, and buffer components (phosphate salts and cations such as magnesium).

These impurities need to be removed from the solution, yet separating sugars from each other in a solution is not trivial. These sugars have similar properties and molecular weights and are hence not easily separated.

### The use of a selective yeast to purify lactose

A solution containing lactose, trehalose, glucose, galactose, UDP-glucose, UDP-galactose, and buffer components and the synthesis enzymes was inoculated with baker's yeast (for instance Bruggeman yeast, *Saccharomyces cerevisiae*). This yeast converts trehalose, glucose, galactose, UDP-glucose, UDP-galactose to biomass and ethanol but leaves the lactose in solution.

After this conversion, the yeast was centrifuged off, leaving in the supernatant lactose, the buffer, and the metabolites formed by *Saccharomyces cerevisiae* (e.g. ethanol, acetate, glycerol) and cell debris. The formation of these metabolites and debris poses a new challenge, these need to be removed. An alternative method for the removal of the yeast is flocculation and decantation or microfiltration with pore size 0.1 to 10µm (ceramic, PES, PVDF membranes).

To this end, the cell debris is removed by ultrafiltration resulting in a solution with less than 100 mg protein per kg dry solid, a DNA content below 10 ng per gram dry solid and an endotoxin was below 10000 EU per gram dry solid. The ultrafiltration was followed by a nanofiltration step in which the buffer salts and yeast metabolites are separated from the lactose. For the nanofiltration a NFX membrane (Synder) with a molecular weight cut-off of 300Da is used at 50°C, which has >99% rejection for lactose. The membrane has a salt rejection <40% which allows easy removal of salts and yeast metabolites while preforming diafiltrations with water. The lactose yield after this step is higher than 95%. During the nanofiltration the solution is further concentrated to a solution of >15% dry matter.

An alternative nanofiltration membrane is NFW (Synder, with molecular weight cut-off <600Da). This membrane has a lactose rejection of >98.5%, salt rejection of <20.0%, which allows easy removal of the salts and yeast metabolites in a diafiltration step. The ash content on total solid after diafiltration is reduced to <5% on total solid.

### The use of a trehalase to selectively hydrolyse trehalose

A solution containing lactose, trehalose, glucose, fructose, galactose, UDP-glucose, UDP-galactose, and buffer components and the synthesis enzymes was treated with a trehalase (α,α-trehalase, Megazymes, approx. 100 mg/l, 40°C, pH 5.5). The residual trehalose is converted during this treatment, while leaving the lactose intact.

After the trehalose hydrolysis, the enzymes are removed by ultrafiltration resulting in a solution with less than 100 mg protein per kg dry solid, a DNA content below 10 ng per gram dry solid and an endotoxin was below 10000 EU per gram dry solid. The ultrafiltration was followed by a nanofiltration step in which the buffer salts and monosaccharides are separated from the lactose. For the nanofiltration a DL series membrane (Suez) with a molecular weight cut-off of 300Da is used at 50°C, which has >99% rejection for lactose but a low rejection for monosaccharides (5-10%). By diafiltration the monosaccharides and the salts are removed in this step. During the nanofiltration the solution is further concentrated to a solution of >15% dry matter. The ash content on total solid after diafiltration is reduced to <5% on total solid.

### Example 26: Demineralization of the lactose solution

The solutions from any of examples 14 to 22 all contain residual salts and nucleotide sugars after treatment. To fully remove the salts from the solution, the solution is treated with a cation and anion exchange resin.

The solutions are first passed through a strong acid cation exchange resin containing column (1L of Amberlite IR120) in the proton form at a temperature of 10°C, resulting in exchange of all cations with a proton in the liquid. The liquid resulting from the cation exchange step was subjected to a weak base anion exchange resin containing column (1L of Amberlite IR400) in the hydroxide form at a temperature of 10°C, exchanging the anions in the liquid for hydroxide ions. After both cation and anion exchange, the pH was set to a pH between 6 and 7. The lactose recovery is between 95 and 98%. The ash content on total solid after ion exchange is reduced to <1% on total solid.

Alternative cation and anion exchange resins are Amberlite IR100, Amberlite IR120, Amberlite FPC22, Dowex 50WX, Finex CS16GC, Finex CS13GC, Finex CS12GC, Finex CS11GC, Lewatit S, Diaion SK, Diaion UBK, Amberjet 1000, Amberjet 1200 and Amberjet 4200, Amberjet 4600, Amberlite IR400, Amberlite IR410, Amberlite IR458, Diaion SA, Diaion UBA120, Lewatit MonoPlus M, Lewatit S7468.

### Example 27: colour removal

To achieve decolourization, several samples throughout the process were subjected to activated charcoal treatment with Norit SX PLUS activated charcoal (0,5% m/v). Colour removal was measured with a spectrophotometer at 420 nm. In all samples the colour intensity at 420nm was reduced 50 to 100 fold. The activated charcoal is filtered of by means of a plate filter or chamber filter press preferably at elevated temperatures.

### Example 28: Concentration

Nanofiltration was carried out with an NF-2540 membrane (DOW) with a cut-off of 200 Da to concentrate the de-ionized solutions after ion exchange, nanofiltation up to 25 Brix or the raw solution after ultrafiltration. During the filtration process a pressure across the membrane in the range of 20-25 bar was used and a process temperature of 45°C. The solution was continuous recirculated over the membrane for concentration, leading to a dry matter content of the concentrate up to 25% Brix.

An alternative method of concentration is falling film evaporation or wiped film evaporation, wherein at high temperature the lactose concentration before drying or crystallization can be increased up to 80%. Typical temperatures are 50-100°C.

### Example 29: Spray drying of enzymatically synthesized lactose

The lactose containing solutions from examples 14 to 28 are spray dried with pilot spray dry equipment. The equipment has an evaporation capacity of 2 kg/h.

For spray drying the liquid was heated to a temperature between 50 and 100°C, to lower the viscosity. The pH of the liquid was set to a pH of 4 to 6. More preferably the pH is set to 4 to 5 and temperatures are kept between 50 and 70°C.

The lactose concentration in the feed is between 5% and 80% brix, depending on if the solution was concentrated (example 14) or treated with nanofiltration (example 11). These concentrations were obtained by rotary evaporation or wiped film evaporation (example 14). The concentrated liquids were fed to the spray dryer at a rate between 50 and 90%. The higher the percentage brix, the faster the feed rate.

The used inlet temperature ranged between 120 and 280°C. The outlet temperature ranged between 100°C and 180°C. The atomizer wheel rotation speed was set between 10000 and 28000 rpm. In one specific test the inlet temperature was set at 184°C, outlet temperature was set at 110°C and atomizer rate was set at 21500 rpm.

The obtained spray dried powder after ultrafiltration, nanofiltration and active charcoal treatment had a white to off white colour and the pH after dissolving in water at a concentration of 10%, is between 4 and 6. The purity of the lactose was above 80% of oligosaccharides on dry solid. The spray dried oligosaccharide mixtures had about 3 to 10% of water content, the protein content was below 100 mg per kg dry solid, the DNA content below 10 ng per gram dry solid and the endotoxin was below 10000 EU per gram dry solid. No GMO DNA could be detected in the powder. The powder that was treated with ion exchange resins has an ash content below 1% (on total dry solid), the Lead content was lower than 0,1 mg/kg dry solid, Arsenic lower than 0,2 mg/kg dry solid, Cadmium lower than 0,1 mg/kg dry solid and Mercury was lower than 0,5 mg/kg dry solid.

### Example 30: Crystallization of lactose

The crystallization process is conducted in an agitated jacketed crystallizer. The concentrated lactose solution from example 28 has a temperature above room temperature, and is cooled to between 20 °C and 25 °C to maximize lactose crystal yield over a period of 12 to 48 h. As the solution is cooled, the lactose crystals crystallized because of the increase in supersaturation. The yield of crystalline lactose in one cycle is about 80%. The mother liquor can be concentrated again by either passing the mother liquor through the nanofiltration step again (to remove concentrated impurities) or concentrated again to a dry matter content of approx. 50%.

### Example 31: Separation of lactose from disaccharides by SMB

For SMB chromatography, a closed loop SMB system equipped with 12 glass columns with the dimensions can be used. Each glass column strong cation ion exchanger resin, typical resins used are Dowex Monosphere in the Ca²⁺ form. The SMB can be run at ambient temperature, but is preferably run above 50°C. The flow rates are adapted over de different zones in the SMB system with a constant feed rate to the SMB. As eluent typically water is used, but food grade ethanol may be used for better separation up to about 10% (v/v).

### Example 32: In vitro synthesis of lactose with purified enzymes

An *E. coli* NiCo21(DE3) strain as described in Example 1 was transformed with an inducible expression plasmid containing either the gene coding for the sucrose synthase (SuSy) from *G*. *max* with UniProt ID P13708, the UDP-glucose-4-epimerase (GalE) from *S. meliloti* with UniProt ID P26503 , the beta-1,4-galactosyltransferase (GalT) from *N. meningitidis* with UniProt ID Q51116or the beta-1,4-galactosyltransferase (GalT) from *P. multocida* with UniProt ID D0EAD4. Expression of the individual enzymes was performed in shake flasks. Cells were harvested and lysed and the enzymes were purified by immobilized metal affinity chromatography (IMAC), as described in Example 1. The purified enzymes (1 mg/mL SuSy, 2 mg/mL GalE and 1.5 mg/mL GalT) were mixed in a one-pot multi-enzyme reaction containing 100 mM sucrose, 25 mM glucose, 10 mM UDP and 10 mM MgCl₂ in 50 mM HEPES buffer (pH 7.0) and incubated for 24 h at 37°C. The formation of lactose was analysed after 24 hours, as described in Example 1. Both combinations of enzymes approached the maximum amount of lactose that can be produced under the specified conditions (Table 2).

**Table 2 Lactose production with different combinations of purified sucrose synthase, UDP-glucose-4-epimerase and beta-1,4-galactosyltransferase (production is normalised to the theoretical maximum amount of lactose that can be produced under the specified conditions)**

| **Sucrose synthase** | **UDP-glucose-4-epimerase** | **Beta-,1,4-galactosyltransferase** | **Lactose production (%)** |
|---|---|---|---|
| *G. max* | *S. meliloti* | *N. meningitidis* | 90 |
| *G. max* | *S. meliloti* | *P. multocida* | 85 |

### Example 33: In vitro synthesis of lactose with purified enzymes

An *E. coli* NiCo21(DE3) strain as described in Example 1 was transformed with an inducible expression plasmid containing either the gene coding for the sucrose synthase (SuSy) from *G*. *max* with UniProt ID P13708, the UDP-glucose-4-epimerase (GalE) from *S. meliloti* with UniProt ID P26503, the glucose isomerase (XylA) from *T. ethanolicus* with UniProt ID D2DK62or the beta-1,4-galactosyltransferase (GalT) from *N. meningitidis* with UniProt ID Q51116. Expression of the individual enzymes was performed in shake flasks. Cells were harvested and lysed and the enzymes were purified by immobilized metal affinity chromatography (IMAC), as described in Example 1. The purified enzymes (2 mg/mL SuSy, 2 mg/mL GalE, 2 mg/mL XylA and 0.4 mg/mL GalT) were mixed in a one-pot multi-enzyme reaction containing 30 mM sucrose, 10 mM UDP and 10 mM MgCl₂ in 50 mM HEPES buffer (pH 7.0), with or without 1 mM CoCl₂, and incubated for 24 h at 30°C. The formation of lactose was analysed, as described in Example 1, and lactose was detected, proving that the combined action of the four enzymes can produce lactose solely from sucrose and a catalytic amount of UDP (Table 3). Addition of Co2+ could moreover enhance the production.

**Table 3 Lactose production with purified sucrose synthase, UDP-glucose-4-epimerase, glucose isomerase and beta-1,4-galactosyltransferase in the presence and absence of Co²⁺ (production normalised to the conditions where no Co²⁺ waspresent in the reaction)**

| **Sucrose synthase** | **UDP-glucose-4-epimerase** | **Glucose isomerase** | **Beta-,1,4-galactosyltransferase** | **Presence of Co²⁺** | **Lactose production (%)** |
|---|---|---|---|---|---|
| *G. max* | *S. meliloti* | *T. ethanolicus* | *N. meningitidis* | No | 100 |
| *G. max* | *S. meliloti* | *T. ethanolicus* | *N. meningitidis* | Yes | 179 |

### Example 34: In vitro synthesis of lactose via cell extract of co-expressed enzymes

An *E. coli* K-12 MG1655 strain as described in Example 1, natively expressing an UDP-glucose-4-epimerase (GalE) with UniProt ID P09147, was transformed with an expression plasmid containing a combination of a constitutive transcriptional unit for a sucrose synthase (SuSy) and a constitutive transcriptional unit for a beta-1,4-galactosyltransferase (GalT). The SuSy was either from *A. caldus* with UniProt ID A0A059ZV61or *G*. *max* with UniProt ID P13708combined with either the GalT from *N. meningitidis* with UniProt ID Q51116 or *P. multocida* with UniProt ID D0EAD4. For the GalT enzymes different transcriptional units (TUs) were evaluated, differing in their promotor, 5'-untranslated region and/or terminator sequence, as described in Example 1. Co-expression of the three enzymes (SuSy, GalE and GalT) in a single strain was performed in a 24-deepwell plate and cells were harvested and lysed, as described in Example 1. Cell lysate from each strain co-expressing the three enzymes was mixed with substrates in a one-pot multi-enzyme reaction containing 100 mM sucrose, 25 mM glucose, 10 mM UDP, 10 mM MgCl₂ and 80% (v/v) cell extract in 50 mM HEPES buffer (pH 7.0) and incubated for 24 h at 30°C. The formation of lactose was analysed as described in Example 1. Multiple combinations of the three co-expressed enzymes lead to the production of lactose (Table 4).

**Table 4 Lactose production with cell extracts of different combinations of sucrose synthase, beta-1,4-galactosyltransferase and UDP-glucose-4-epimerase, co-expressed in a single strain (production normalised to the production of the G. max SuSy / P. multocida GalT (TU variant 2) combination)**

| **Sucrose synthase** | **UDP-glucose-4-epimerase** | **Beta-,1,4-galactosyltransferase** | **Lactose production (%)** |
|---|---|---|---|
| *G. max* | *E. coli* | *N. meningitidis* (TU variant 1) | 198 |
| *G. max* | *E. coli* | *N. meningitidis* (TU variant 2) | 194 |
| *G. max* | *E. coli* | *N. meningitidis* (TU variant 3) | 163 |
| *G. max* | *E. coli* | *P. multocida* (TU variant 1) | 150 |
| *G. max* | *E. coli* | *P. multocida* (TU variant 2) | 100 |
| *G. max* | *E. coli* | *P. multocida* (TU variant 3) | 161 |
| *A. caldus* | *E. coli* | *N. meningitidis* (TU variant 3) | 137 |
| *A. caldus* | *E. coli* | *P. multocida* (TU variant 3) | 165 |
| *A. caldus* | *E. coli* | *P. multocida* (TU variant 4) | 156 |

### Example 35: In vitro synthesis of lactose via cell extract of co-expressed enzymes

An *E. coli* K-12 MG1655 strain as described in Example 1, natively expressing an UDP-glucose-4-epimerase (GalE) with UniProt ID P09147, was transformed with an expression plasmid containing a combination of a constitutive transcriptional unit for a sucrose synthase (SuSy) from *G. max* with UniProt ID P13708, a constitutive transcriptional unit for a glucose isomerase (XylA) from *T. ethanolicus* with UniProt ID D2DK62 and a constitutive transcriptional unit for a beta-1,4-galactosylstransferase (GalT) from either *N*. *meningitidis* with UniProt ID Q51116 or *P. multocida* with UniProt ID D0EAD4. For the GalT enzymes different transcriptional units (TUs) were evaluated, differing in their promotor, 5'-untranslated region and/or terminator sequence, as described in Example 1. Co-expression of the four enzymes (SuSy, GalE, XylA and GalT) in a single strain was performed in a 24-deepwell plate and cells were harvested and lysed, as described in Example 1. Cell lysate from each strain co-expressing the four enzymes was mixed with substrates in a one-pot multi-enzyme reaction containing 100 mM sucrose, 10 mM UDP, 10 mM MgCl₂ and 80% (v/v) cell extract in 50 mM HEPES buffer (pH 7.0) and incubated for 24 h at 30°C. The formation of lactose was analysed as described in Example 1. Multiple combinations of the four co-expressed enzymes lead to the production of lactose (Table 5).

**Table 5 Lactose production with cell extracts of different combinations of sucrose synthase, beta-1,4-galactosyltransferase, glucose isomerase and UDP-glucose-4-epimerase, co-expressed in a single strain (production normalised to the production of the T. ethanolicus XylA (TU variant 1) / P. multocida GalT (TU variant 1) combination)**

| **Sucrose synthase** | **UDP-glucose-4-epimerase** | **Glucose isomerase** | **Beta-,1,4-galactosyltransferase** | **Lactose production (%)** |
|---|---|---|---|---|
| *G. max* | *E. coli* | *T. ethanolicus* (TU variant 1) | *N. meningitidis* (TU variant 1) | 108 |
| *G. max* | *E. coli* | *T. ethanolicus* (TU variant 1) | *P. multocida* (TU variant 1) | 100 |
| *G. max* | *E. coli* | *T. ethanolicus* (TU variant 1) | *P. multocida* (TU variant 2) | 136 |
| *G. max* | *E. coli* | *T. ethanolicus* (TU variant 2) | *N. meningitidis* (TU variant 1) | 117 |
| *G. max* | *E. coli* | *T. ethanolicus* (TU variant 2) | *N. meningitidis* (TU variant 2) | 153 |
| *G. max* | *E. coli* | *T. ethanolicus* (TU variant 2) | *P. multocida* (TU variant 1) | 105 |
| *G. max* | *E. coli* | *T. ethanolicus* (TU variant 2) | *P. multocida* (TU variant 2) | 117 |

### Example 36: Whole cell biotransformation synthesis of lactose

An *E. coli* K-12 MG1655 strain as described in Example 1, natively expressing an UDP-glucose-4-epimerase (GalE) with UniProt ID P09147, was transformed with an expression plasmid containing a combination of a constitutive transcriptional unit for a sucrose synthase (SuSy) and a constitutive transcriptional unit for a beta-1,4-galactosylstransferase (GalT). The SuSy was either from *A. caldus* with UniProt ID A0A059ZV61 or *G*. *max* with UniProt ID P13708 combined with either the GalT from *N. meningitidis* with UniProt ID Q51116 or *P. multocida* with UniProt ID D0EAD4. For the SuSy from *G*. *max* and the GalT enzymes different transcriptional units (TUs) were evaluated, differing in their promotor, 5'-untranslated region and/or terminator sequence, as described in Example 1. Co-expression of the enzymes was performed in a 24-deepwell plate and cells were harvested and permeabilised by one freeze-thaw cycle, as described in Example 1. Whole cell biotransformation was performed by incubating 10 mg/mL cells, 100 mM sucrose, 25 mM glucose, 10 mM UDP and 5 mM MgCl₂ in 25 mM phosphate buffer (pH 7) and incubated for 48 h at 30°C. The formation of lactose was analysed as described in Example 1. Multiple combinations of the three co-expressed enzymes lead to the production of lactose in a whole cell biotransformation setup (Table 6).

**Table 6 Lactose production in a whole cell biotransformation with a strain expressing different combinations of sucrose synthase, beta-1,4-galactosyltransferase and UDP-glucose-4-epimerase (production normalised to the production of the G. max SuSy (TU variant 2) / P. multocida GalT (TU variant 1) combination)**

| **Sucrose synthase** | **UDP-glucose-4-epimerase** | **Beta-,1,4-galactosyltransferase** | **Lactose production (%)** |
|---|---|---|---|
| *G. max* (TU variant 1) | *E. coli* | *N. meningitidis* (TU variant 1) | 222 |
| *G. max* (TU variant 1) | *E. coli* | *N. meningitidis* (TU variant 2) | 384 |
| *G. max* (TU variant 1) | *E. coli* | *N. meningitidis* (TU variant 3) | 203 |
| *G. max* (TU variant 1) | *E. coli* | *P. multocida* (TU variant 1) | 373 |
| *G. max* (TU variant 1) | *E. coli* | *P. multocida* (TU variant 2) | 151 |
| *G. max* (TU variant 2) | *E. coli* | *P. multocida* (TU variant 1) | 100 |
| *G. max* (TU variant 2) | *E. coli* | *P. multocida* (TU variant 2) | 152 |
| *A. caldus* | *E. coli* | *N. meningitidis* (TU variant 2) | 442 |
| *A. caldus* | *E. coli* | *N. meningitidis* (TU variant 3) | 379 |
| *A. caldus* | *E. coli* | *P. multocida* (TU variant 1) | 216 |
| *A. caldus* | *E. coli* | *P. multocida* (TU variant 2) | 287 |
| *A. caldus* | *E. coli* | *P. multocida* (TU variant 3) | 206 |

### Example 37: Whole cell biotransformation synthesis of lactose

An *E. coli* K-12 MG1655 strain as described in Example 1, natively expressing an UDP-glucose-4-epimerase (GalE) with UniProt ID P09147, was transformed with an expression plasmid containing a combination of a constitutive transcriptional unit for a sucrose synthase (SuSy) and a constitutive transcriptional unit for a beta-1,4-galactosylstransferase (GalT). The SuSy was either from *A. caldus* with UniProt ID A0A059ZV61 or *G*. *max* with UniProt ID P13708 combined with either the GalT from *N. meningitidis* with UniProt ID Q51116 or *P. multocida* with UniProt ID D0EAD4. For the SuSy from *G*. *max* and the GalT enzymes different transcriptional units (TUs) were evaluated, differing in their promotor, 5'-untranslated region and/or terminator sequence, as described in Example 1. The novel strains were evaluated in a growth experiment for production of lactose according to the culture conditions provided in Example 1 in which the strains were cultivated in minimal medium with 30 g/L sucrose, 10 g/L glucose and 10 g/L glycerol. The strains were grown in four biological replicates in a 96-well plate. After 72h of incubation, the culture broth was harvested and the formation of lactose was analysed as described in Example 1. Multiple combinations of the three co-expressed enzymes lead to the production of lactose in this whole cell biotransformation setup without permeabilisation step (Table 7).

**Table 7 Lactose production in a whole cell biotransformation with a strain expressing different combinations of sucrose synthase, beta-1,4-galactosyltransferase and UDP-glucose-4-epimerase (production normalised to the production of the A. caldus SuSy / N. meningitidis (TU variant 1) combination)**

| **Sucrose synthase** | **UDP-glucose-4-epimerase** | **Beta-,1,4-galactosyltransferase** | **Lactose production (%)** |
|---|---|---|---|
| *G. max* (TU variant 1) | *E. coli* | *N. meningitidis* (TU variant 1) | 242 |
| *G. max* (TU variant 1) | *E. coli* | *N. meningitidis* (TU variant 2) | 149 |
| *G. max* (TU variant 1) | *E. coli* | *N. meningitidis* (TU variant 3) | 1828 |
| *G. max* (TU variant 1) | *E. coli* | *N. meningitidis* (TU variant 4) | 1687 |
| *G. max* (TU variant 1) | *E. coli* | *P. multocida* (TU variant 1) | 1907 |
| *G. max* (TU variant 1) | *E. coli* | *P. multocida* (TU variant 2) | 655 |
| *G. max* (TU variant 1) | *E. coli* | *P. multocida* (TU variant 3) | 2017 |
| *G. max* (TU variant 1) | *E. coli* | *P. multocida* (TU variant 4) | 1824 |
| *G. max* (TU variant 2) | *E. coli* | *P. multocida* (TU variant 1) | 1006 |
| *G. max* (TU variant 2) | *E. coli* | *P. multocida* (TU variant 2) | 276 |
| *G. max* (TU variant 2) | *E. coli* | *P. multocida* (TU variant 3) | 378 |
| *G. max* (TU variant 2) | *E. coli* | *P. multocida* (TU variant 4) | 1106 |
| *A. caldus* | *E. coli* | *N. meningitidis* (TU variant 1) | 100 |
| *A. caldus* | *E. coli* | *N. meningitidis* (TU variant 2) | 104 |
| *A. caldus* | *E. coli* | *N. meningitidis* (TU variant 3) | 793 |
| *A. caldus* | *E. coli* | *N. meningitidis* (TU variant 4) | 746 |
| *A. caldus* | *E. coli* | *P. multocida* (TU variant 1) | 873 |
| *A. caldus* | *E. coli* | *P. multocida* (TU variant 2) | 306 |
| *A. caldus* | *E. coli* | *P. multocida* (TU variant 3) | 928 |
| *A. caldus* | *E. coli* | *P. multocida* (TU variant 4) | 806 |

### Example 38: Whole cell biotransformation synthesis of lactose in a bioreactor

An *E. coli* K-12 MG1655 strain as described in Example 1, natively expressing an UDP-glucose-4-epimerase (GalE) with UniProt ID P09147, was transformed with an expression plasmid containing a constitutive transcriptional unit for a sucrose synthase (SuSy) from *G. max* with UniProt ID P13708 and a constitutive transcriptional unit for a beta-1,4-galactosylstransferase (GalT) from *N. meningitidis* with UniProt ID Q51116. The resulting strain was subsequently evaluated at bioreactor scale in a fed-batch process, as described in Example 1. Glycerol, sucrose and glucose were added in the batch medium and sucrose and glucose were added during the fed-batch phase via an additional feed. The formation of lactose at the end of the process was analysed as described in Example 1 and compared to the formation in a microtiter plate. The titer was almost 12 times as high in a bioreactor as compared to a microtiter plate (Table 8).

**Table 8 Lactose production in a whole cell biotransformation with a strain expressing a sucrose synthase, beta-1,4-galactosyltransferase and UDP-glucose-4-epimerase (production in a bioreactor normalised to the production in a microtiter plate)**

| **Format** | **Titer (%)** |
|---|---|
| Microtiter plate | 100 |
| Bioreactor | 1192 |

## Claims

1. A method for the production of lactose, **characterised in that** said method comprises use of i) a synthase and ii) UDP, for forming UDP-glucose, wherein said method is a fermentation process, a bioconversion, or an enzymatic process **characterised in that** said synthase is a sucrose synthase, said synthase forms fructose and said method further comprising use of an UDP-glucose-4-epimerase and use of a beta 1,4-galactosyltransferase; and when said method is i) an enzymatic process the reaction is performed between 50°C and 80°C, or ii) a fermentation process or a bioconversion a) 1) the enzymatic conversions are performed by at least one enzyme produced by a cell synthesizing said enzyme; or 2) said lactose is produced by a cell genetically engineered to produce all enzymes needed for production of lactose; and/or b) the fermentation process or bioconversion process is performed at a temperature between about 25°C and about 50°C .

2. The method according to claim 1, wherein said UDP-glucose-4-epimerase converts said UDP-glucose to UDP-galactose.

3. The method according to claim 2, wherein said UDP-galactose is a galactose donor for said beta 1,4-galactosyltransferase to form lactose, wherein preferably said beta 1,4-galactosyltransferase is a lactose synthase.

4. The method according to any one of claims 1 to 3, wherein said beta 1,4-galactosyltransferase converts glucose with UDP-galactose to form lactose and UDP, wherein preferably said beta 1,4-galactosyltransferase is a lactose synthase.

5. The method according to any one of claims 1 to 4, wherein the UDP released by the beta 1,4-galactosyltransferase is recycled to be used by the sucrose synthase to form UDP-glucose.

6. The method according to any one of claims 1 to 5, wherein said beta 1,4-galactosyltransferase uses glucose as an acceptor and UDP-galactose as a donor, wherein preferably said beta 1,4-galactosyltransferase is a lactose synthase.

7. The method according to any one of claims 1 to 6, wherein said sucrose synthase forms a fructose and said fructose is converted to glucose.

8. The method according to any one of claims 1 to 7, wherein said method is an enzymatic process and wherein i) at least one of said enzymes are immobilised and/or ii) the reaction is performed between 55°C and 70°C.

9. The method according to any one of claims 1 to 7, wherein said method is a fermentation process or a bioconversion **characterised in that** the fermentation process or bioconversion process is performed at a temperature between about 25°C and about 40°C.

10. The method according to any of one of the previous claims, wherein the carbon source used for conversion into lactose is chosen from the list of sucrose or combination of sucrose and glucose.

11. The method according to claim 10 for enzymatic production of lactose using i) sucrose or ii) sucrose and glucose as a carbon source, **characterized in that** lactose is produced by using i) sucrose or ii) sucrose and glucose as a carbon source, and the reaction thereof is catalysed by a plurality of enzyme combinations.

12. The method according to claim 10 using sucrose synthase, and wherein
a) said carbon source is sucrose, wherein i) sucrose is converted with said sucrose synthase into UDP-glucose and fructose, ii) said UDP-glucose is converted into UDP-galactose and iii) fructose is converted into glucose and iv) said UDP-galactose from step ii) and said glucose from step iii) are converted into lactose; or
b) said carbon source is a combination of sucrose and glucose, wherein i) sucrose is converted with said sucrose synthase into UDP-glucose and fructose, ii) said UDP-glucose is converted into UDP-galactose and iii) said UDP-galactose from step ii) and said glucose which was provided together with the sucrose are converted into lactose.

13. The method according to any one of the previous claims, wherein said method further comprises a step of purification of said lactose, preferably said lactose is purified by at least one of the following purification steps: microfiltration, centrifugation, ultrafiltration, nanofiltration, ion exchange , a cation exchange, an anion exchange, Simulated Moving Bed (SMB), colour removal, removal of the carbon source, removal of sucrose or glucose by addition of yeast, removal of sucrose by addition of an invertase.

14. The method according to any one of the previous claims, wherein said method comprises a step of drying and/or crystallisation of said lactose, preferably said crystals are dried, preferably said step of drying or drying of crystals comprises any one or more of spray drying, lyophilization, evaporation, precipitation, spray freeze drying, freeze spray drying, band drying, belt drying, vacuum band drying, vacuum belt drying, drum drying, roller drying, vacuum drum drying, vacuum roller drying, and agitated thin film drying.

## Patentansprüche

1. Verfahren zur Herstellung von Lactose, **dadurch gekennzeichnet, dass** das Verfahren die Verwendung von i) einer Synthase und ii) UDP zur Bildung von UDP-Glucose umfasst, wobei es sich bei dem Verfahren um einen Fermentationsprozess, eine Biokonversion oder einen enzymatischen Prozess handelt, **dadurch gekennzeichnet, dass** die Synthase eine Saccharose-Synthase ist, die Synthase Fructose bildet und wobei das Verfahren ferner die Verwendung einer UDP-Glucose-4-Epimerase und die Verwendung einer Beta-1,4-Galactosyltransferase umfasst, und, wenn es sich bei dem Verfahren um i) ein enzymatisches Verfahren handelt, die Reaktion zwischen 50 °C und 80 °C durchgeführt wird, oder um ii) einen Fermentationsprozess oder eine Biokonversion handelt, a) 1) die enzymatischen Umwandlungen von mindestens einem Enzym durchgeführt werden, das von einer das Enzym synthetisierenden Zelle produziert wird, oder 2) die Lactose von einer Zelle produziert wird, die gentechnisch verändert ist, so dass sie alle Enzyme produziert, die zur Herstellung von Lactose benötigt werden, und/oder b) der Fermentations- oder Biokonversionsprozess bei einer Temperatur zwischen etwa 25 °C und etwa 50 °C durchgeführt wird.

2. Verfahren nach Anspruch 1, wobei die UDP-Glucose-4-Epimerase die UDP-Glucose in UDP-Galactose umwandelt.

3. Verfahren nach Anspruch 2, wobei die UDP-Galactose als Galactose-Donor dient, so dass die Beta-1,4-Galactosyltransferase Lactose bildet, wobei es sich bei der Beta-1,4-Galactosyltransferase bevorzugt um eine Lactose-Synthase handelt.

4. Verfahren nach einem der Ansprüche 1 bis 3, wobei die Beta-1,4-Galactosyltransferase Glucose mit UDP-Galactose unter Bildung von Lactose und UDP umwandelt, wobei es sich bei der Beta-1,4-Galactosyltransferase bevorzugt um eine Lactose-Synthase handelt.

5. Verfahren nach einem der Ansprüche 1 bis 4, wobei das durch die Beta-1,4-Galactosyltransferase freigesetzte UDP rückgeführt wird, um von der Saccharose-Synthase zur Bildung von UDP-Glucose verwendet zu werden.

6. Verfahren nach einem der Ansprüche 1 bis 5, wobei die Beta-1,4-Galactosyltransferase Glucose als Akzeptor und UDP-Galactose als Donor verwendet, wobei es sich bei der Beta-1,4-Galactosyltransferase bevorzugt um eine Lactose-Synthase handelt.

7. Verfahren nach einem der Ansprüche 1 bis 6, wobei die Saccharose-Synthase eine Fructose bildet und die Fructose in Glucose umgewandelt wird.

8. Verfahren nach einem der Ansprüche 1 bis 7, wobei es sich bei dem Verfahren um einen enzymatischen Prozess handelt und wobei i) mindestens eines der Enzyme immobilisiert ist und/oder ii) die Reaktion zwischen 55 °C und 70 °C durchgeführt wird.

9. Verfahren nach einem der Ansprüche 1 bis 7, wobei es sich bei dem Verfahren um einen Fermentationsprozess oder eine Biokonversion handelt, **dadurch gekennzeichnet, dass** der Fermentations- oder Biokonversionsprozess bei einer Temperatur zwischen etwa 25 °C und etwa 40 °C durchgeführt wird.

10. Verfahren nach einem der vorherigen Ansprüche, wobei die zur Umwandlung in Lactose verwendete Kohlenstoffquelle aus der Liste von Saccharose oder einer Kombination von Saccharose und Glucose gewählt wird.

11. Verfahren nach Anspruch 10 zur enzymatischen Herstellung von Lactose unter Verwendung von i) Saccharose oder ii) Saccharose und Glucose als Kohlenstoffquelle, **dadurch gekennzeichnet, dass** Lactose durch Verwendung von i) Saccharose oder ii) Saccharose und Glucose als Kohlenstoffquelle hergestellt und die Umsetzung davon durch mehrere Enzymkombinationen katalysiert wird.

12. Verfahren nach Anspruch 10 unter Verwendung von Saccharose-Synthase, und wobei
a) die Kohlenstoffquelle Saccharose ist, wobei i) Saccharose mit der Saccharose-Synthase in UDP-Glucose und Fructose umgewandelt wird, ii) die UDP-Glucose in UDP-Galactose umgewandelt wird und iii) Fructose in Glucose umgewandelt wird und iv) die UDP-Galactose aus Schritt ii) und die Glucose aus Schritt iii) in Lactose umgewandelt werden; oder
b) die Kohlenstoffquelle eine Kombination von Saccharose und Glucose ist, wobei i) Saccharose mit der Saccharose-Synthase in UDP-Glucose und Fructose umgewandelt wird, ii) die UDP-Glucose in UDP-Galactose umgewandelt wird und iii) die UDP-Galactose aus Schritt ii) und die Glucose, die zusammen mit der Saccharose bereitgestellt wurde, in Lactose umgewandelt werden.

13. Verfahren nach einem der vorherigen Ansprüche, wobei das Verfahren ferner einen Schritt der Aufreinigung der Lactose umfasst, wobei die Lactose bevorzugt durch mindestens einen der folgenden Aufreinigungsschritte aufgereinigt wird: Mikrofiltration, Zentrifugation, Ultrafiltration, Nanofiltration, Ionenaustausch, ein Kationenaustausch, ein Anionenaustausch, SMB (Simulated Moving Bed), Farbentfernung, Entfernung der Kohlenstoffquelle, Entfernung von Saccharose oder Glucose durch Zugabe von Hefe, Entfernung von Saccharose durch Zugabe einer Invertase.

14. Verfahren nach einem der vorherigen Ansprüche, wobei das Verfahren einen Schritt umfasst, bei dem die Lactose getrocknet und/oder kristallisiert wird, wobei die Kristalle bevorzugt getrocknet werden, wobei der Trocknungs- bzw. Kristalltrocknungsschritt bevorzugt eine oder mehrere von Sprühtrocknung, Lyophilisierung, Verdampfung, Fällung, Sprühgefriertrocknung, Gefriersprühtrocknung, Bandtrocknung, Transportbandtrocknung, Vakuum-Bandtrocknung, Vakuum-Transportbandtrocknung, Trommeltrocknung, Walzentrocknung, Vakuum-Trommeltrocknung, Vakuum-Walzentrocknung und Turbulenz-Dünnschichttrocknung umfasst.

## Revendications

1. Procédé de production de lactose, **caractérisé en ce que** ledit procédé comprend l'utilisation de i) une synthase et ii) UDP, pour former du UDP-glucose, ledit procédé étant un processus de fermentation, une bioconversion, ou un processus enzymatique **caractérisé en ce que** ladite synthase est une saccharose synthase, ladite synthase forme du fructose et ledit procédé comprenant en outre l'utilisation d'une UDP-glucose-4-épimérase et l'utilisation d'une bêta-1,4-galactosyltransférase ; et quand ledit procédé est i) un processus enzymatique, la réaction est réalisée entre 50 °C et 80 °C, ou ii) un processus de fermentation ou une bioconversion a) 1) les conversions enzymatiques sont effectuées par au moins une enzyme produite par une cellule synthétisant ladite enzyme ; ou 2) ledit lactose est produit par une cellule génétiquement modifiée pour produire toutes les enzymes nécessaires pour la production de lactose ; et/ou b) le processus de fermentation ou le processus de bioconversion est réalisé à une température comprise entre environ 25 °C et environ 50 °C.

2. Procédé selon la revendication 1, dans lequel ladite UDP-glucose-4-épimérase convertit ledit UDP-glucose en UDP-galactose.

3. Procédé selon la revendication 2, dans lequel ledit UDP-galactose est un donneur de galactose pour ladite bêta-1,4-galactosyltransférase pour former du lactose, dans lequel de préférence ladite bêta-1,4-galactosyltransférase est une lactose synthase.

4. Procédé selon l'une quelconque des revendications 1 à 3, dans lequel ladite bêta-1, 4-galactosyltransférase convertit le glucose avec UDP-galactose pour former du lactose et UDP, dans lequel de préférence ladite bêta-1,4-galactosyltransférase est une lactose synthase.

5. Procédé selon l'une quelconque des revendications 1 à 4, dans lequel l'UDP libéré par la bêta-1,4-galactosyltransférase est recyclé pour être utilisé par la saccharose synthase pour former l'UDP-glucose.

6. Procédé selon l'une quelconque des revendications 1 à 5, dans lequel ladite bêta-1, 4-galactosyltransférase utilise le glucose comme un accepteur et l'UDP-galactose comme un donneur, dans lequel de préférence ladite bêta-1,4-galactosyltransférase est une lactose synthase.

7. Procédé selon l'une quelconque des revendications 1 à 6, dans lequel ladite saccharose synthase forme un fructose et ledit fructose est converti en glucose.

8. Procédé selon l'une quelconque des revendications 1 à 7, dans lequel ledit procédé est un processus enzymatique et dans lequel i) au moins une desdites enzymes est immobilisée et/ou ii) la réaction est effectuée entre 55 °C et 70 °C.

9. Procédé selon l'une quelconque des revendications 1 à 7, dans lequel ledit procédé est un processus de fermentation ou une bioconversion, caractérisé(e) en ce que le processus de fermentation ou le processus de bioconversion est réalisé à une température comprise entre environ 25 °C et environ 40 °C.

10. Procédé selon l'une quelconque des revendications précédentes, dans lequel la source de carbone utilisée pour la conversion en lactose est choisie parmi la liste du saccharose ou une combinaison de saccharose et de glucose.

11. Procédé selon la revendication 10 pour la production enzymatique de lactose utilisant i) du saccharose ou ii) du saccharose et du glucose en tant que source de carbone, **caractérisé en ce que** le lactose est produit en utilisant i) du saccharose ou ii) du saccharose et du glucose en tant que source de carbone, et la réaction correspondante est catalysée par une pluralité de combinaisons enzymatiques.

12. Procédé selon la revendication 10 utilisant une saccharose synthase, et dans lequel
a) ladite source de carbone est le saccharose, dans lequel i) le saccharose est converti avec ladite saccharose synthase en UDP-glucose et en fructose, ii) ledit UDP-glucose est converti en UDP-galactose et iii) le fructose est converti en glucose et iv) ledit UDP-galactose de l'étape ii) et ledit glucose de l'étape iii) est converti en lactose ; ou
b) ladite source de carbone est une combinaison de saccharose et de glucose, dans lequel i) le saccharose est converti avec ladite saccharose synthase en UDP-glucose et en fructose, ii) ledit UDP-glucose est converti en UDP-galactose et iii) ledit UDP-galactose de l'étape ii) et ledit glucose qui a été fourni conjointement avec le saccharose sont convertis en lactose.

13. Procédé selon l'une quelconque des revendications précédentes, dans lequel ledit procédé comprend en outre une étape de purification dudit lactose, de préférence ledit lactose est purifié par au moins une des étapes de purification suivantes : microfiltration, centrifugation, ultrafiltration, nanofiltration, échange d'ions, échange de cations, échange d'anions, lit mobile simulé (SMB), élimination de couleur, élimination de la source de carbone, élimination de saccharose ou de glucose par ajout de levure, élimination de saccharose par ajout d'une invertase.

14. Procédé selon l'une quelconque des revendications précédentes, dans lequel ledit procédé comprend une étape de séchage et/ou de cristallisation dudit lactose, de préférence lesdits cristaux sont séchés, de préférence ladite étape de séchage ou de séchage de cristaux comprend l'un ou plusieurs parmi le séchage par pulvérisation, la lyophilisation, l'évaporation, la précipitation, la lyophilisation par pulvérisation, le séchage par pulvérisation et lyophilisation, le séchage à bande, le séchage à courroie, le séchage à bande sous vide, le séchage à courroie sous vide, le séchage au tambour, le séchage au rouleau, le séchage au tambour sous vide, le séchage au rouleau sous vide, et le séchage en film mince agité.
